(19) Europäisches Patentamt / European Patent Office / Office européen des brevets

(11) **EP 4 125 376 B1**

(12) **EUROPEAN PATENT SPECIFICATION**

(45) Date of publication and mention
of the grant of the patent:
**15.07.2026 Bulletin 2026/29**

(21) Application number: **21719966.0**

(22) Date of filing: **26.03.2021**

(51) International Patent Classification (IPC):
*A01P 1/00* (2006.01)     *A01N 25/34* (2006.01)
*A01N 37/46* (2006.01)     *C07K 7/06* (2006.01)

(52) Cooperative Patent Classification (CPC):
**A01N 37/46; A01N 25/34; A01P 1/00; C07K 7/06;**
Y02A 50/30

(86) International application number:
**PCT/IB2021/052519**

(87) International publication number:
**WO 2021/191851 (30.09.2021 Gazette 2021/39)**

(54) **ANTIMICROBIAL PEPTIDES**

ANTIMIKROBIELLE PEPTIDE

PEPTIDES ANTIMICROBIENS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.03.2020 IT 202000006511**

(43) Date of publication of application:
**08.02.2023 Bulletin 2023/06**

(73) Proprietor: **Materias S.r.l.**
**80122 Napoli (IT)**

(72) Inventors:
• **PALMIERI, Gianna**
**80078 Pozzuoli (Napoli) (IT)**
• **BALESTRIERI, Marco**
**80070 Ischia (Napoli) (IT)**
• **NICOLAIS, Luigi**
**80056 Ercolano (Napoli) (IT)**

(74) Representative: **Comoglio, Elena et al**
**Jacobacci & Partners S.p.A.**
**Corso Emilia 8**
**10152 Torino (IT)**

(56) References cited:
**WO-A1-2015/038339     WO-A1-2019/012158**

• **BRUNA AGRILLO ET AL: "Functionalized
Polymeric Materials with Bio-Derived
Antimicrobial Peptides for "Active" Packaging",
INTERNATIONAL JOURNAL OF MOLECULAR
SCIENCES, vol. 20, no. 3, 30 January 2019
(2019-01-30), pages 601, XP055755659, DOI:
10.3390/ijms20030601**

Remarks:
The complete document including Reference
Table(s) and the Sequence Listing(s) can be
downloaded from the EPO website

## Description

### FILED OF THE INVENTION

The present invention relates to peptides with bactericidal activity and the use thereof as antimicrobial agents.

### STATE OF THE ART

Treatment of bacterial infections with antibiotics is one of the pillars of human medicine. Unfortunately, the effectiveness of antibiotics has become limited due to increased bacterial resistance and decreased success in the search for new antibiotics. Today, infectious diseases are the second leading cause of death worldwide and the largest cause of premature deaths and loss of work productivity in industrialized countries.

A major limitation to the development of antibiotics is the difficulty of finding new compounds having properties equivalent to conventional antibiotics, namely low host toxicity and broad spectrum of action against bacterial pathogens.

A promising group of compounds are antimicrobial peptides, i.e., a group of molecules active in the innate immune response that constitutes the first line of defence against pathogens (Alberts B, Johnson A, Lewis J, et al. Innate immunity, in Molecular Biology of the Cell. 4th edition. New York: Garland Science; 2002). These molecules are also referred to as AMPs ("antimicrobial peptides") and are produced in many tissues and cell types of organisms such as plants, insects, amphibians and higher organisms. Their amino acid composition and structure-related chemical-physical characteristics allow them to interact selectively with the lipid bilayer of the bacterial membrane, thereby causing the death of microorganisms. Antimicrobial peptides appear to have a high potential for activity on many bacterial strains pathogenic for humans, both Gram negative and Gram positive; furthermore, these peptides, unlike the drugs currently in use, do not easily select mutants and do not induce antibiotic-resistance phenomena; finally, they exhibit a synergistic effect with conventional antibiotics and can often activate the host's innate immune response without showing immunogenicity.

In recent years, numerous antimicrobial peptides have been identified through various techniques ranging from *in silico* analysis to the screening of peptide libraries.

In particular, patent application WO2015038339 describes the structure of 753 7-12-amino acid peptides, which are indicated as having an anti-biofilm and/or immunomodulatory activity. Some of the peptides described therein are tested for their ability to inhibit biofilms respectively formed by *Klebsiella pneumoniae, Staphylococcus aureus, E. coli, Acinetobacter baumannii, Salmonella enterica ssp.* Typhimurium, *Burkholderia cenocepacia.* In this context, the decapeptide named HE10 is described, which has the sequence VRLIVRIWRR (SEQ ID NO: 33).

Patent application WO2019012158 discloses the use of some antimicrobial peptides, including IDR-1018-K6, as bacterial agents for the prevention and/or treatment of contamination of a product or surface by a specific bacterium, *Listeria monocytogenes;* this document further discloses the use of the peptides in the treatment of infections caused in a subject by *Listeria monocytogenes.*

To date, the studies carried out and the amount of data available in numerous databases (Wang, G., Li, X. and Wang, Z. (2016) APD3: the antimicrobial peptide database as a tool for research and education, Nucleic Acids Research 44, D1087-D1093) show that antimicrobial peptides can have different structures and target a variety of microorganisms. Furthermore, in many cases, the peptides tend to assume the structure with which the activity is associated only after contact with cell membranes. Based on the presence or absence of two key elements of the secondary structure ($\alpha$-helix and $\beta$-sheet), AMPs are commonly classified into four main classes: (i) peptides with linear $\alpha$-helical structure, which represent the largest and best studied group; (ii) peptides with linear extended structures (devoid of $\alpha$-helix or $\beta$-sheet elements); (iii) peptides containing $\beta$-sheets and (iv) peptides containing $\alpha$-helix and $\beta$-sheet elements. Hundreds of different sequences have been identified from natural sources and a multitude of analogues and synthetic derivatives have been produced, whose size and diversity are constantly expanding (Johannes Koehbach and David J. Craik. The Vast Structural Diversity of Antimicrobial Peptides, Trends in Pharmacological Sciences, July 2019, Vol. 40, No. 7). Therefore, it appears that, to date, there is no clear structure/function relationship for this category of antimicrobial molecules, and therefore it is extremely difficult to predict their activity.

Most of the known AMPs have small dimensions, generally ranging from 12 to 50 amino acid residues. The longer ones, such as Nisin (34 aa) or LL37 (37 aa), are at least partially structured, also thanks to the longer amino acid chain thereof. In contrast, the shorter ones have a lower percentage probability of being structured and it is therefore equally difficult to predict their activity (Ralf Mikut, Serge Ruden, Markus Reischl, Frank Breitling, Rudolf Volkmer, Kai Hilpert. Improving short antimicrobial peptides despite elusive rules for activity Biochimica et Biophysica Acta 1858 (2016) 1024-1033).

Although the antibacterial action of Nisin has been known for decades, its mechanism of action still requires further investigation. This may be due to the fact that Nisin exerts its action through different mechanisms depending on the structural properties of the membranes of the target bacteria. Nisin itself is only active against Gram positive bacteria, however its combination with treatments disrupting the cell membrane makes it also active against Gram negative bacteria (Sukrita Punyauppa-path, Parichat Phumkhachorn, Pongsak Rattanachaikunsopon NISIN: PRODUCTION AND MECHANISM OF ANTIMICROBIAL ACTION, Int J Cur Res Rev | Vol 7 • Issue 2 • January 2015). Several studies show that some structural Nisin motifs are essential for the formation of the "pore" in bacterial membranes, while other portions of the peptide contribute to the bactericidal action by inhibiting cell wall synthesis. This shows that Nisin has at least two different mechanisms of action against bacteria (H Brotz and HG Sahl New insights into the mechanism of action of lantibiotics - diverse biological effects by binding to the same molecular target, Journal of Antimicrobial Chemotherapy, 2000, 46 1-6).

LL-37 also modulates its mechanism of action based on the structure of the different lipids that form the cell membrane: it induces the formation of pores in the unsaturated phospholipid bilayers and interferes with membrane functions in the presence of saturated phospholipids, producing fibrous peptide-lipid superstructures rich in $\alpha$-helix structures. (Mahdi Shahmiri, Marta Enciso, Christopher G. Adda, Brian J. Smith, Matthew A. Perugini & Adam Mechler Membrane Core-Specific Antimicrobial Action of Cathelicidin LL-37 Peptide Switches Between Pore and Nanofibre Formation, Scientific Reports volume 6, Article number: 38184 (2016)).

It is therefore clear that larger peptides may generally have greater folding variability and complexity, in other words they are more likely to assume various secondary structures even in different portions of the amino acid chain. Therefore, it is extremely difficult to understand and control the mechanisms by which a peptide with a longer amino acid sequence is able to carry out its biological activity, since the variables that can affect and/or impair it are more numerous. Furthermore, in case of use of a peptide for the functionalization of a polymeric surface through a covalent bond, a longer amino acid sequence would statistically increase the sites with which the peptide can be bound to the material of interest (PET; PVC; PL; etc.), making the conformations that the molecule can assume after forming the bond, (or the bonds at several points of the sequence), and consequently also the possibility of retaining the antimicrobial activity, unpredictable.

On the other hand, small molecules lend themselves better to be designed and/or modified with the aim of retaining or amplifying a specific activity. In addition, the small size of an antimicrobial peptide significantly reduces the costs for the synthesis and purification thereof.

For years, the main goal of scientific research has been to evaluate the efficacy and therapeutic potential of AMPs against bacterial infections. However, recent studies have shown that AMPs, for example those belonging to the cathelicidin and defensin family, can be particularly promising therapeutic tools even against emerging infectious viral pathogens for which there are currently no approved vaccines or treatments [Human Antimicrobial Peptides as Therapeutics for Viral Infections, Viruses 2019, 11, 704].

In light of the above, it is clear that there is a need to identify new antimicrobial peptides having bactericidal capacity on a broad spectrum of microorganisms, including Gram negative and Gram positive bacteria, fungi and yeasts, and possibly also having antiviral action. A further need is that the aforementioned antimicrobial peptides have an amino acid chain of moderate length, possibly shorter than that of the currently known antimicrobial peptides. Yet another need is that the costs for the synthesis and purification of the aforementioned peptides are as low as possible.

## SUMMARY OF THE INVENTION

These and other needs have been met by the present invention, which provides a new series of antimicrobial peptides having basic, net charge and hydrophobic characteristics similar to those of the antimicrobial peptides known so far, but a relatively short amino acid sequence and a broad spectrum of activities. The antimicrobial peptides of the present invention are characterized by a specific sequence which distinguishes them from other known antimicrobial peptides, and by a broad spectrum of bactericidal activity, as well as by an effective antimicrobial activity against fungi and yeasts. The antimicrobial peptides of the present invention are also likely to exhibit antiviral activity, since molecules belonging to cationic peptide categories, to which the peptides of the invention belong, have shown antiviral properties, in particular towards both DNA and RNA pericapsidic viruses. The antiviral activity often appears to be related to the inhibition of processes of virus adsorption and entry into cells or is the result of a direct effect on the viral pericapsid, while some peptides that are internalized can affect cellular antiviral mechanisms and block the expression of viral genes.

The antimicrobial peptides of the present invention consist of the amino acid sequence represented by the following general formula:

$$X_1LX_2WVX_3IWX_4X_5,$$

wherein $X_1$, $X_2$, $X_3$, $X_4$ and $X_5$ are independently selected from K and R and wherein each amino acid is independently in the D or L configuration.

Salts and solvates of the aforementioned peptides also fall within the scope of the invention.

Example 1 in the following experimental part shows the synthesis of the peptide designated as RiLK1, falling within the general formula above, which is representative of the new series of AMP peptides forming the object of the invention. The RiLK1 peptide and all the other AMP peptides of the invention share the fact that they contain the structural motif W-X-X-X-W and have similar values of hydrophobicity, hydropathicity, amphiphilicity, hydrophilicity, net charge, Boman index and propensity for a disordered conformation, which are indicative of similar bactericidal properties (see Examples 13 and 14).

Example 2 assessed the maximum solubility of the RiLK1 peptide with all amino acids in the D configuration or in the L configuration in three different solvents, namely, ultrapure water, DMSO and Dulbecco's Phosphate Buffered Saline (DPBS), verifying that the peptide, both in the L configuration and in the D configuration, dissolves completely up to a concentration $\leqq$ 10 mg/mL in the three conditions examined.

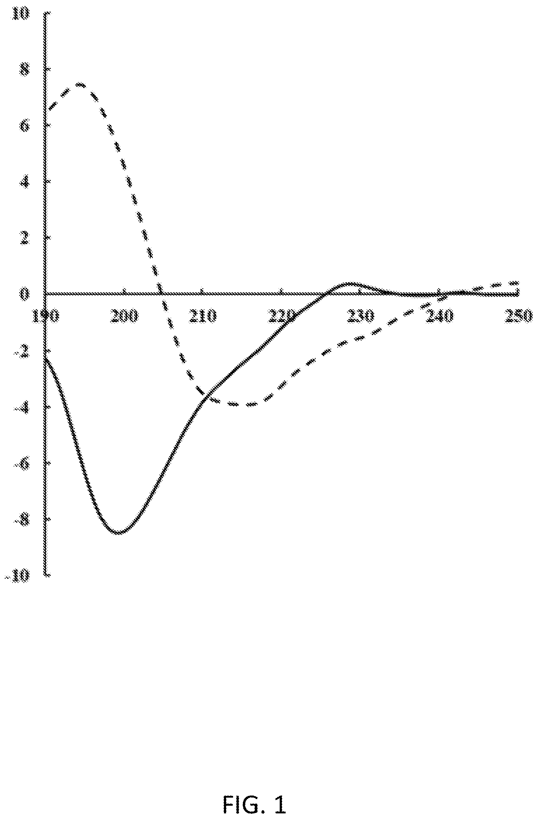

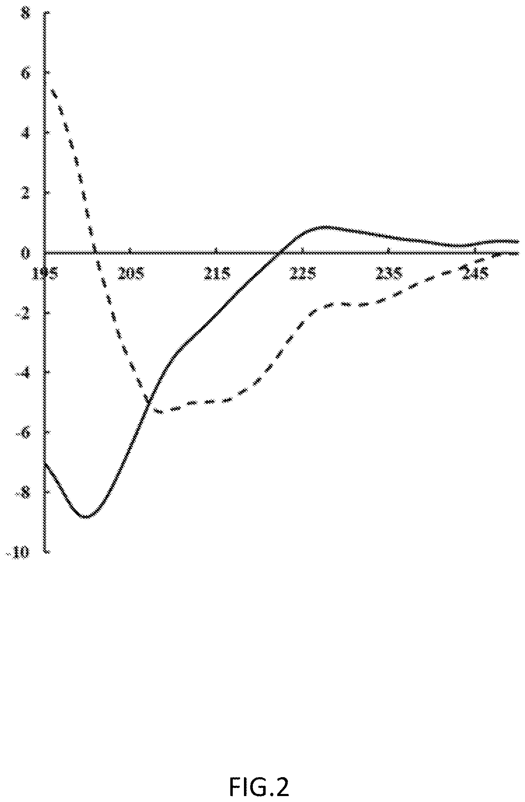

In Example 3, the RiLK1 peptide was dissolved in water (Figure 1) or in a sodium acetate buffer solution at pH 4 (Figure 2) in the presence or absence of SDS. The different solutions were analysed by Circular Dichroism (CD) spectroscopy. The addition of SDS to the solution is a condition that mimics the presence of bacterial membranes. This analysis shows that the peptide is unstructured (solid black line) but that in the presence of SDS (dashed black line) it is structured, assuming a mixed $\alpha$-helix/$\beta$-sheet conformation, wherein the contribution of one or of the other element of the secondary structure varies according to the different environmental conditions (in this case the pH).

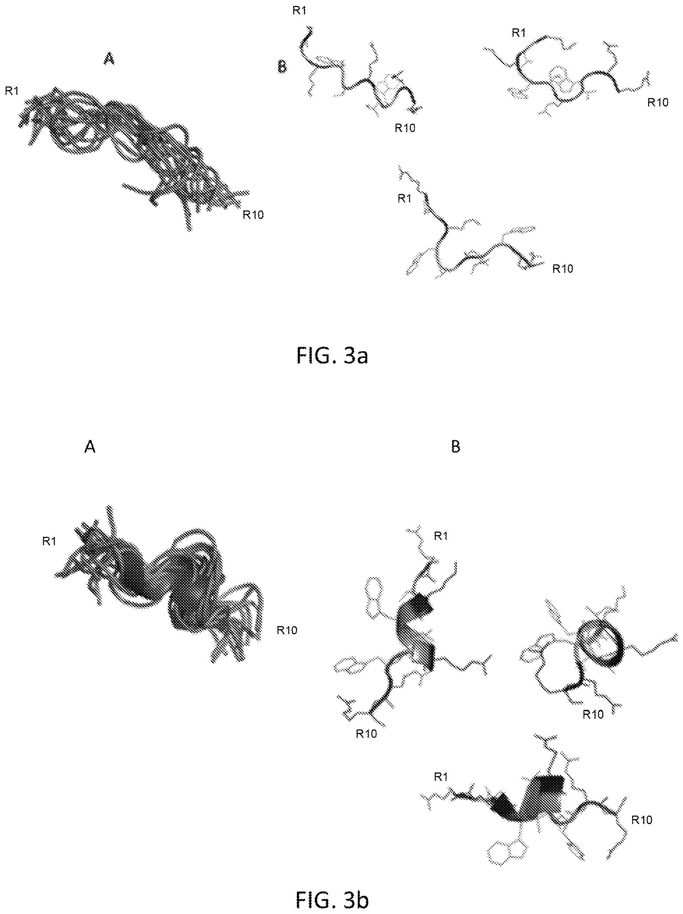

A further structural analysis of the RiLK1 peptide was carried out by Nuclear Magnetic Resonance (NMR) spectroscopy, a particularly effective technique for the structural characterization of organic molecules, which made it possible to draw three-dimensional models of the RiLK1 peptide of the present invention. These results confirm that the peptide tends to structure itself only in the presence of a micellar solution of SDS (Figure 3).

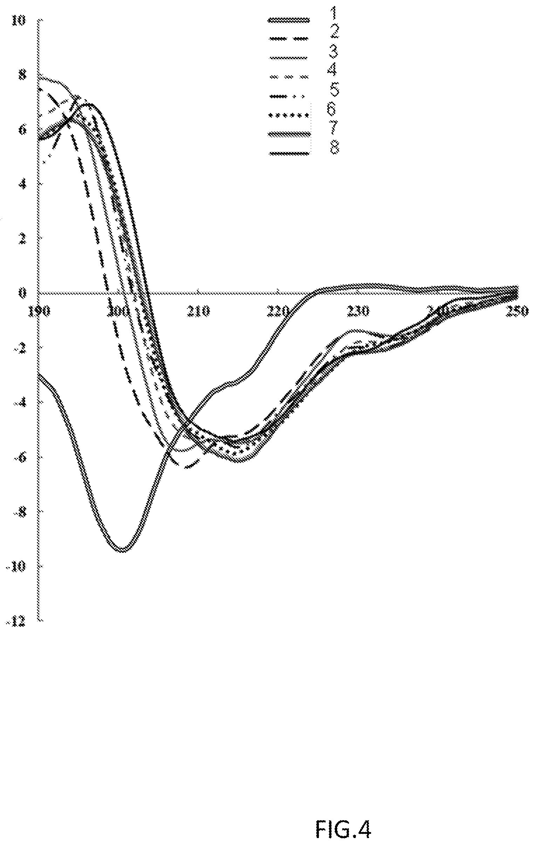

In Example 4, the kinetics of the structural rearrangements of the peptide, monitored over 24 hours by circular dichroism (CD) spectroscopy, show that the mixed $\alpha$-helix/$\beta$-sheet conformation is maintained and that the secondary structure $\beta$-sheet element is predominant over the $\alpha$-helix (Figure 4-5).

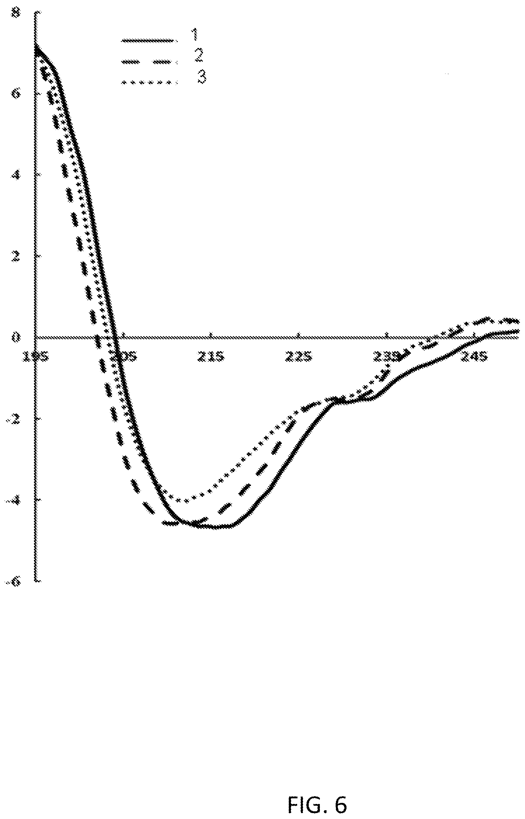

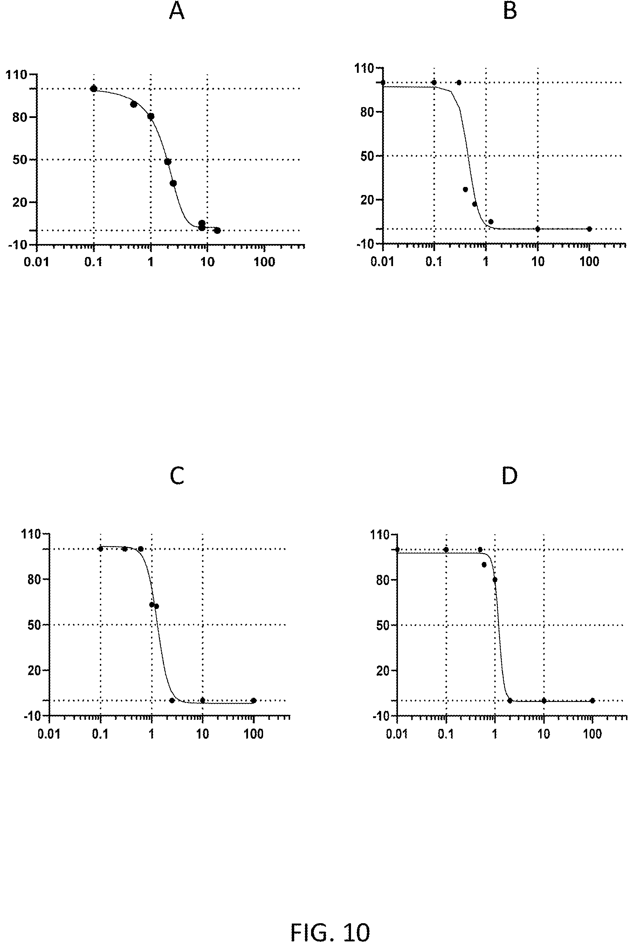

Examples 5 to 8 show that the RiLK1 peptide of the present invention is stable at different temperatures, at different pH values, and in possible conditions of use such as, for example, at high salt concentration (Figures 6-7-8-9). Therefore, it is very interesting to note that the peptide according to the invention, characterized by the presence of only 10 aa, has a high bactericidal activity. As shown in Example 9, RiLK1 has in fact a powerful bactericidal activity both towards Gram positive bacteria, such as S. *aureus* and L. monocytogenes, and towards Gram negative bacteria, such as *S. thyphimurium* and *E. coli* (Figures 10-11).

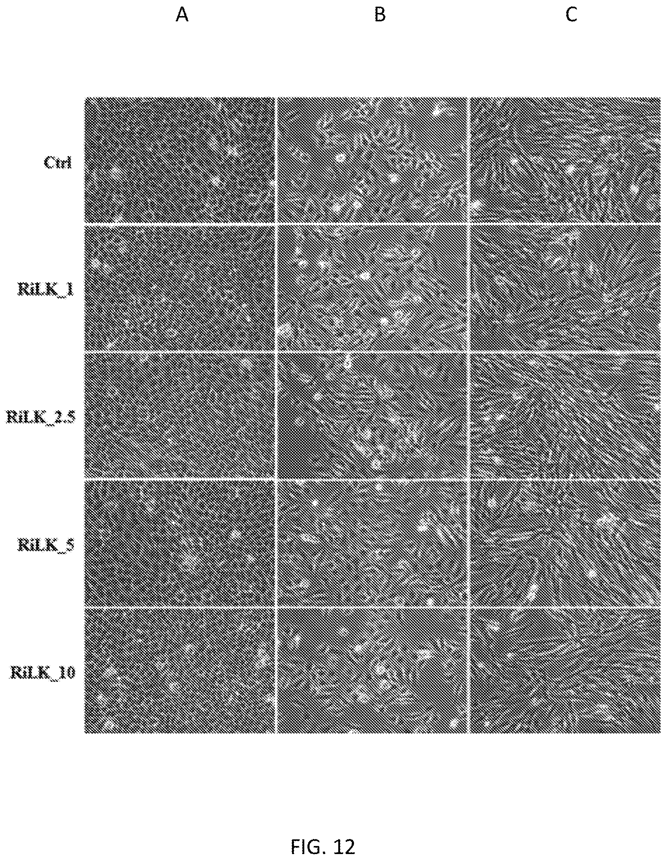

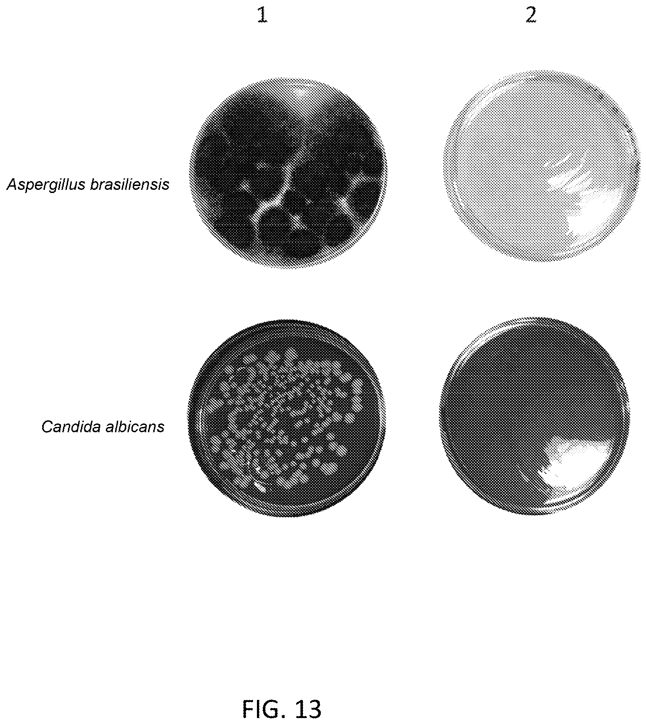

Example 10 shows that the RiLK1 peptide is not cytotoxic towards a panel of human cell lines (Figure 12) and Example 11 shows that it also has an efficient activity against fungi and yeasts, such as *Aspergillus brasiliensis* and *Candida albicans* (Figure 13).

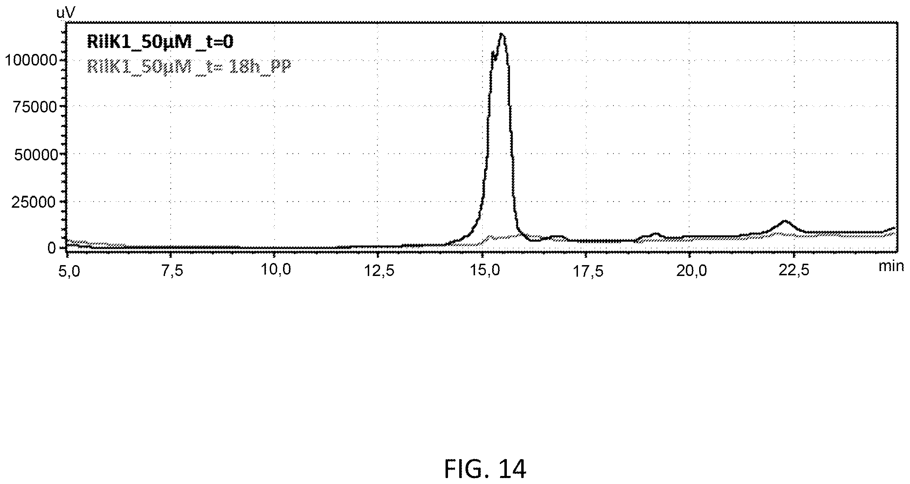

Finally, in Example 12 the RiLK1 peptide was covalently linked to a polymeric surface with high reaction yields (Figure 14) and the polymer thus functionalized was shown to have high stability in terms of peptide release and a preservative activity aimed at increasing food shelf life, significantly reducing the proliferation of mesophilic bacteria and yeasts in a dairy product contacted therewith.

An antimicrobial peptide as defined by the general formula above, for use as a medicament, also falls within the scope of the invention.

A specific therapeutic application of the antimicrobial peptides of the invention relates to the therapeutic treatment of an infection caused by Gram negative bacteria, Gram positive bacteria, fungi, yeasts and/or viruses.

Also within the scope of the invention is a pharmaceutical formulation comprising an antimicrobial peptide as defined by the general formula above and at least one pharmaceutically acceptable excipient and/or carrier.

Consumers' attention to the possible effects on their health related to food quality is having a great impact on food processing and preservation industries. Compared to the past, today the consumer is increasingly aware and attentive to the quantity and quality of chemical preservatives in food and to the effectiveness of both conventional preservation methods and innovative preservation technologies in extending the shelf life and improving the safety of a wide range of food products, while preserving the taste/flavour thereof. Several microorganisms are recognized today as responsible for food infections related to food consumption; *Listeria monocytogenes,* a Gram-positive bacterium causing listeriosis, and *Salmonella typhimurium,* a Gram-negative bacterium causing salmonellosis, are certainly among these. These microorganisms can grow in a wide variety of foods, usually raw foods, such as undercooked meat, raw vegetables, fish products, cheese prepared with unpasteurized milk, or ready-to-use food products that are industrially processed and require low temperature preservation. Foodborne listeriosis is a rather rare but serious disease, with a mortality rate (20-30%) comparable or higher to that of other foodborne diseases, such as, for example, salmonellosis.

Therefore, the present invention also includes the use of an antimicrobial peptide as defined by the general formula above, as an antimicrobial agent for the prevention of contamination of an article by and/or for the decontamination of an article (e.g., a food, a material, a container or a tool) from Gram negative bacteria, Gram positive bacteria, fungi, yeast and/or viruses.

An antimicrobial composition comprising an antimicrobial peptide as defined by the general formula above and at least one carrier is also included within the scope of the invention.

Finally, the invention relates to an article, such as, for example, a food, a material, a container or a tool, bearing an antimicrobial peptide covalently bonded to a surface thereof, or bearing an antimicrobial peptide contained in a coating adhered to said surface, wherein said antimicrobial peptide is as defined by the general formula above.

**BRIEF DESCRIPTION OF THE FIGURES**

Figure 1 and Figure 2 show the Circular Dichroism (CD) spectra of the RiLK1 peptide obtained in water and sodium acetate buffer at pH 4, respectively, in the absence (solid black line) or in the presence (dashed black line) of 3mM SDS, as described in Example 2. The abscissa axis shows the wavelength (nm), the ordinate axis shows the average molar ellipticity value per residue x $10^{-3}$, expressed as degrees $cm^2/dmol^{-1}$.

Figure 3a and Figure 3b show RiLK1 peptide structures obtained by $^1$H NMR in $H_2O$ and in a 150mM micellar solution of SDS, respectively. Figure 3a shows a disordered structure compatible with a structurally undefined and random (random coil) conformation of the peptide (A, B). Figure 3b shows that the presence of SDS induces an iso-orientation of the side chains with the formation of a central $\alpha$-helix structure (A, B). R1 and R10, respectively, represent the starting and ending amino acid of the RiLK1 peptide.

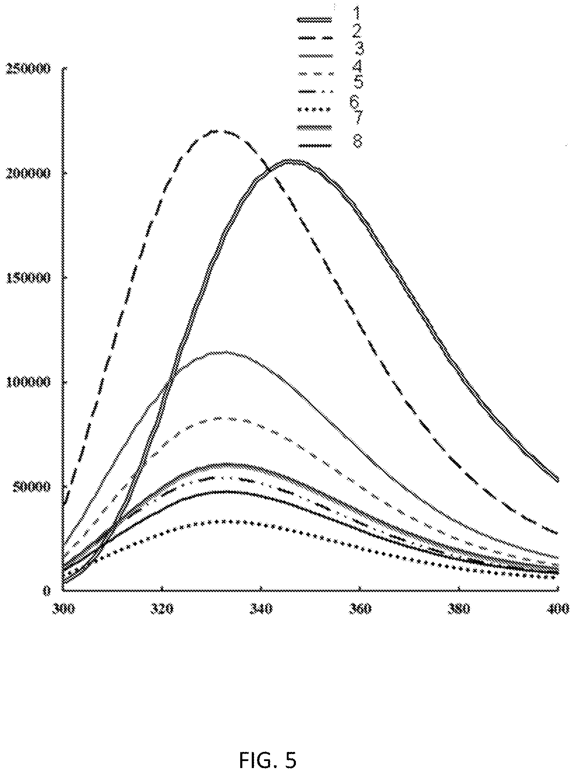

Figure 4 and Figure 5, respectively, show the Circular Dichroism (CD) and fluorescence spectra of the RiLK1 peptide obtained in the absence (line 1) or in the presence (lines 2-8) of 3mM SDS by monitoring the sample for 24 hours (line 2 at time point = 0; line 3 at time point = 5min; line 4 at time point = 30min; line 5 at time point = 1 hour; line 6 at time point = 2 hours; line 7 at time point = 5 hours; line 8 at time point = 24 hours) to highlight the kinetics of the structural rearrangements as described in Example 3. In Figure 4, the abscissa axis shows the wavelength (nm), the ordinate axis shows the average molar ellipticity value per residue x $10^{-3}$, expressed as degrees $cm^2/dmol^{-1}$. In Figure 5, the abscissa axis shows the wavelength (nm), the ordinate axis shows the fluorescence intensity value.

Figure 6 shows the Circular Dichroism (CD) spectrum of the RiLK1 peptide obtained in Tris HCL at pH 7 and in the presence of 3mM SDS by monitoring the sample for 24 hours (line 1 at time point = 0; line 2 at 4°C and time point = 24 hours; line 3 at 25°C and time point = 24 hours), as described in Example 4. The abscissa axis shows the wavelength (nm), the ordinate axis shows the average molar ellipticity value per residue x $10^{-3}$, expressed as degrees $cm^2/dmol^{-1}$.

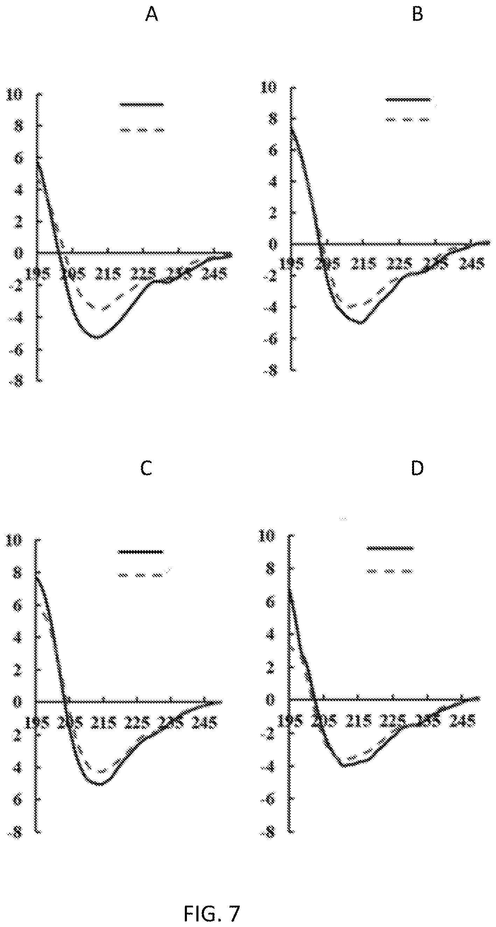

Figure 7 shows the Circular Dichroism (CD) spectra of the RiLK1 peptide obtained by monitoring the sample for 24 hours (solid black line at time point = 0; dashed black line at time point = 24 hours) in solutions at different pHs, pH 2 (A), pH 4 (B), pH 7 (C) and pH 9 (D), respectively, as described in Example 5. The abscissa axis shows the wavelength (nm), the ordinate axis shows the average molar ellipticity value per residue x $10^{-3}$, expressed as degrees $cm^2/dmol^{-1}$.

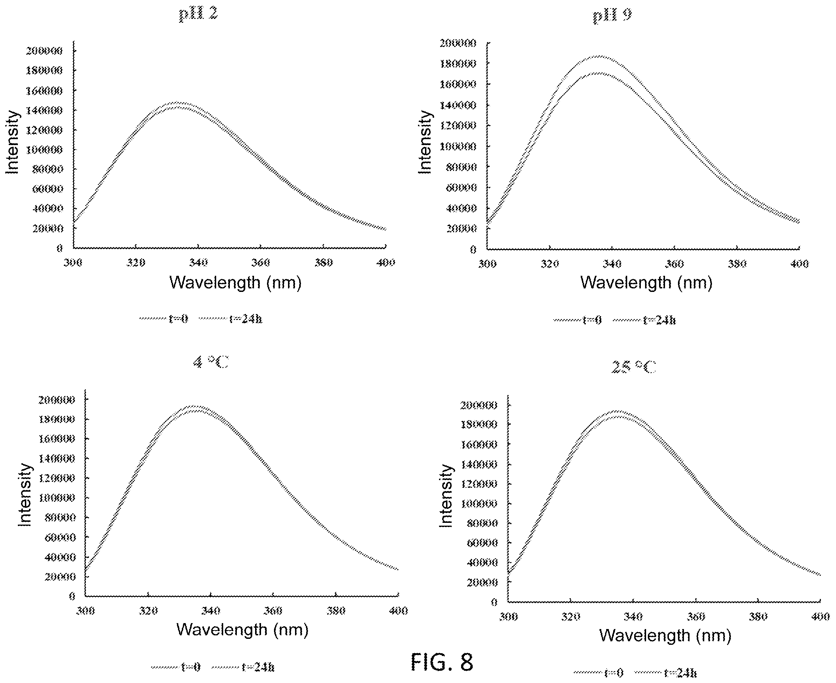

Figure 8 shows the fluorescence spectra of the RiLK1 peptide recorded in the presence of a micellar solution of SDS (150 mM) by monitoring the sample for up to 24 hours in solutions at different pHs, pH 2 (A) and pH 9 (B), respectively, and at different incubation temperatures, 4°C and 25°C, respectively, to highlight the stability thereof, as described in Example 6. In the figure, the abscissa axis shows the wavelength (nm), the ordinate axis shows the fluorescence intensity value.

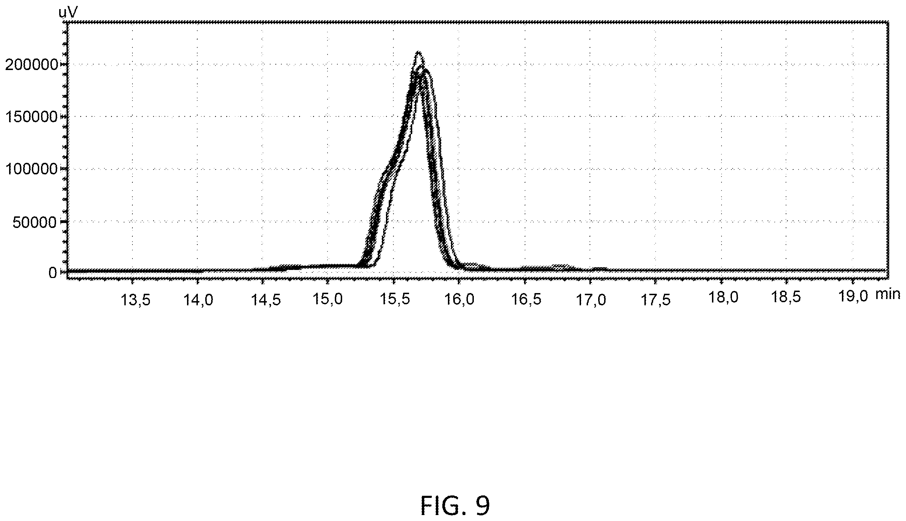

Figure 9 shows the overlapping of chromatograms obtained by RP-HPLC chromatography performed on RiLK1 peptide samples incubated in the presence of high salt concentration (1 M NaCl) and monitored for 9 days (lines 1-8 time points 0; 5h; 24h; 29h; 48h; 53h; 6d; 9d), as described in Example 7.

Figure 10 shows dose-response curves obtained with the RiLK1 peptide against S. *aureus* (A), *L. monocytogenes* (B), *S. thyphimurium* (C) and *E. coli* (D); the abscissa axis shows the peptide concentrations ($\mu$M), the ordinate axis shows the % of surviving cells, as described in Example 8a.

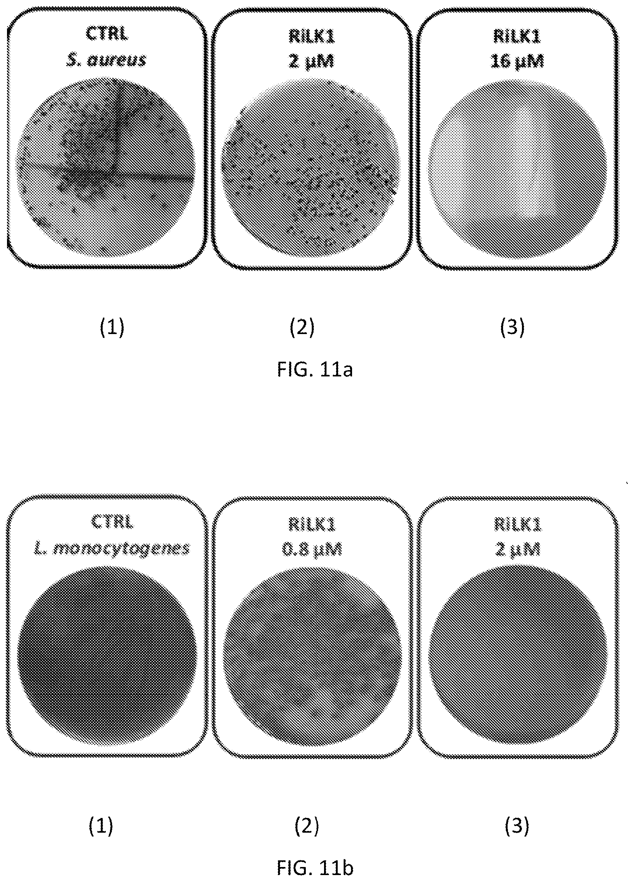

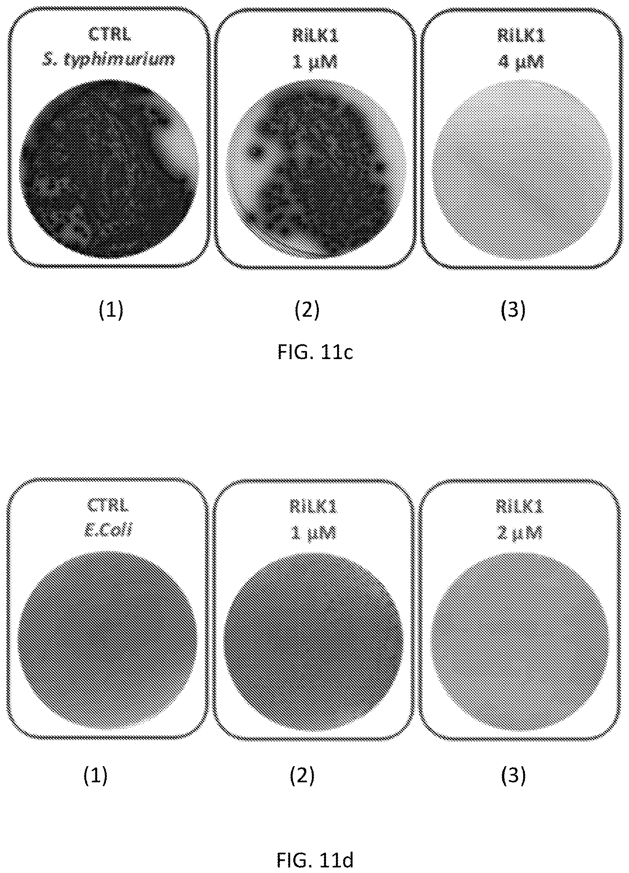

Figures 11a, 11b, 11c and 11d show for each bacterium tested, i.e., *S. aureus, L. monocytogenes, S. thyphimurium* and *E. coli,* respectively, photographic images of a control plate incubated with the bacterium without the addition of the RiLK1 peptide (1), a plate incubated with the bacterium and treated with a RiLK1 peptide concentration lower than its MBC (2), a plate incubated with the bacterium and treated with a RiLK1 peptide concentration corresponding to its MBC (3), as described in Example 8b.

In Figure 12, columns A, B and C, respectively, show photographic images of a control plate with HaCat, WI38 and TIG3-20 cells incubated for 24 hours in the absence of the peptide (Ctrl), and in the presence of a RiLK1 peptide concentration of 1 $\mu$M (RiLK_1), 2.5 $\mu$M (RiLK_2.5), 5 $\mu$M (RiLK_5) and 10 $\mu$M (RiLK_10).

Figure 13a shows photographic images acquired after 7 days of growth of a control plate incubated with the fungus *Aspergillus brasiliensis* in the absence of the RiLK1 peptide (1) and of a plate incubated with the fungus treated for 6 h with the RiLK1 peptide at a concentration of 25 µM corresponding to its Minimal Fungicidal Concentration MFC (2), as described in Example 10.

Figure 13b shows photographic images acquired after 7 days of growth of a control plate incubated with the yeast *Candida albicans* in the absence of the RiLK1 peptide (1) and of a plate incubated with the fungus treated for 6 h with the RiLK1 peptide at a concentration of 25 µM corresponding to its MFC (2), as described in Example 10.

Figure 14 shows chromatographic profiles obtained by reversed phase high performance liquid chromatography (RP-HPLC) following the procedure for the 24-hour corona functionalization of pre-activated polypropylene (PP) discs with the RiLK1 peptide at a concentration of 50 µM. The immobilization yield was calculated by measuring the ratio between the peptide peak areas at time point t = 24h and time point t = 0.

## DETAILED DESCRIPTION OF THE INVENTION

A first object of the present invention is an antimicrobial peptide consisting of the amino acid sequence represented by the following general formula:

$$X_1LX_2WVX_3IWX_4X_5$$

wherein $X_1$, $X_2$, $X_3$, $X_4$ and $X_5$ are independently selected from K and R and wherein each amino acid is independently in the D or L configuration,
or a salt or a solvate thereof.

It should be noted that all amino acid sequences are represented in the present description by formulas whose orientation from left to right is in the conventional direction, i.e., from the amino terminus to the carboxyl terminus.
According to a preferred embodiment, the amino acids in the above general formula are all in the D configuration or in the L configuration.
According to another preferred embodiment, in the above general formula, at least one of $X_1$, $X_3$, $X_4$ and $X_5$ has the meaning of R; more preferably, $X_4$ and $X_5$ have the meaning of R; still more preferably, $X_3$, $X_4$ and $X_5$ have the meaning of R; even more preferably, $X_1$, $X_3$, $X_4$ and $X_5$ have the meaning of R.
In another preferred embodiment, $X_2$ has the meaning of K.
In another preferred embodiment, at least one of $X_1$, $X_3$, $X_4$ and $X_5$ has the meaning of R and $X_2$ has the meaning of K; more preferably, $X_4$ and $X_5$ have the meaning of R and $X_2$ has the meaning of K; still more preferably, $X_3$, $X_4$ and $X_5$ have the meaning of R and $X_2$ has the meaning of K; even more preferably, $X_1$, $X_3$, $X_4$ and $X_5$ have the meaning of R and $X_2$ has the meaning of K.
The following amino acid sequences are particularly preferred:
$RLX_2WVRIWX_4X_5$, $RLX_2WVRIWRX_5$, $RLX_2WVRIWX_4K$, $RLKX_2WVRIWKK$, $X_1LKWVX_3IWRR$, $X_1LKWVRIWRR$, $RLKWVX_3IWRR$, $X_1LRWVX_3IWKK$, $X_1LRWVKIWKK$, $KLRWVX_3IWKK$,
wherein $X_1$, $X_2$, $X_3$, $X_4$ and $X_5$ are independently selected from K and R and wherein each amino acid is independently in the D or L configuration.
The following specific amino acid sequences are further preferred:
RLKWVRIWRR (SEQ ID NO: 1), KLRWVRIWRR (SEQ ID NO: 2), RLRWVRIWRR (SEQ ID NO: 3), KLKWVRIWRR (SEQ ID NO: 4), RLKWVKIWRR (SEQ ID NO: 5), KLRWVKIWRR (SEQ ID NO: 6), RLRWVKIWRR (SEQ ID NO: 7), KLKWVKIWRR (SEQ ID NO: 8), RLKWVRIWKR (SEQ ID NO: 9), KLRWVRIWKR (SEQ ID NO: 10), RLRWVRIWKR (SEQ ID NO: 11), KLKWVRIWKR (SEQ ID NO: 12), RLKWVKIWKR (SEQ ID NO: 13), KLRWVKIWKR (SEQ ID NO: 14), RLRWVKIWKR (SEQ ID NO: 15), KLKWVKIWKR (SEQ ID NO: 16), RLKWVRIWRK (SEQ ID NO: 17), KLRWVRIWRK (SEQ ID NO: 18), RLRWVRIWRK (SEQ ID NO: 19), KLKWVRIWRK (SEQ ID NO: 20), RLKWVKIWRK (SEQ ID NO: 21), KLRWVKIWRK (SEQ ID NO: 22), RLRWVKIWRK (SEQ ID NO: 23), KLKWVKIWRK (SEQ ID NO: 24), RLKWVRIWKK (SEQ ID NO: 25), KLRWVRIWKK (SEQ ID NO: 26), RLRWVRIWKK (SEQ ID NO: 27), KLKWVRIWKK (SEQ ID NO: 28), RLKWVKIWKK (SEQ ID NO: 29), KLRWVKIWKK (SEQ ID NO: 30), RLRWVKIWKK (SEQ ID NO: 31), KLKWVKIWKK (SEQ ID NO: 32),
wherein each amino acid is independently in the D or L configuration.
In the context of the present invention, the term "solvate" or "solvates" refers to complexes of the peptides of the invention with the solvents in which the synthesis reaction takes place or in which they are precipitated or crystallized. For example, a complex with water is known as a "hydrate".
Salts and solvates suitable for the purposes of the invention are those that do not lead to a change in the conformation or stability of the peptides according to the invention, and therefore, do not interfere with their biological activity.

Preferred salts according to the invention are pharmaceutically acceptable salts that do not lead to a change in the conformation or stability of the peptides according to the invention. By way of illustrative and non-limiting examples, pharmaceutically acceptable acid addition salts include those formed with the hydrochloric, hydrobromic, acetic, phosphoric, lactic, pyruvic, acetic, trifluoroacetic, succinic, perchloric, fumaric, maleic, glycolic, lactic, salicylic, oxaloacetic, methanesulfonic, ethanesulfonic, p-toluenesulfonic, formic, benzoic, malonic, naphthalene-2-sulfonic, benzenesulfonic and isethionic acids. Other acids, such as oxalic acid, although not pharmaceutically acceptable per se, can be useful as intermediates for obtaining the peptides of the invention and pharmaceutically acceptable salts thereof. Acceptable base salts include ammonium salts, alkali metal salts, for example potassium and sodium salts, alkaline earth metal salts, for example calcium and magnesium salts, and salts with organic bases, for example dicyclohexylamine and N-methyl-D-glucosamine.

A second object of the present invention is an antimicrobial peptide consisting of the amino acid sequence represented by the following general formula:

$$X_1LX_2WVX_3IWX_4X_5$$

wherein $X_1$, $X_2$, $X_3$, $X_4$ and $X_5$ are independently selected from K and R and wherein each amino acid is independently in the D or L configuration,
or a salt or a solvate thereof,
for use as a medicament.

A third object of the present invention is an antimicrobial peptide consisting of the amino acid sequence represented by the following general formula:

$$X_1LX_2WVX_3IWX_4X_5$$

wherein $X_1$, $X_2$, $X_3$, $X_4$ and $X_5$ are independently selected from K and R and wherein each amino acid is independently in the D or L configuration,
or a salt or a solvate thereof,
for use in the therapeutic treatment of an infection caused by Gram negative bacteria, Gram positive bacteria, yeasts, fungi and/or viruses.

Within the scope of the invention, the Gram negative bacterium is preferably selected from Campylobacter such as, for example, *Campylobacter coli, Campylobacter concisus, Campylobacter jejuni, Campylobacter C. rectus; Arcobacter* such as, for example, *Arcobacter butzleri, Arcobacter cryaerophilus; Citrobacter* such as, for example, *Citrobacter amalonaticus, Citrobacter braakii, Citrobacter farmeri, Citrobacter freundii, Citrobacter gillenii, Citrobacter koseri; Enterobacter* such as, for example, *Enterobacter aerogenes, Enterobacter agglomerans, Enterobacter cloacae, Enterobacter cowanii, Enterobacter gergoviae; Escherichia* such as, for example, *Escherichia coli; Klebsiella; Morganella* such as, for example, *Morganella Morganii; Proteus* such as, for example, *Proteus vulgaris, Proteus mirabilis; Shigella* such as, for example, *Shigella dysenteriae; Salmonella* such as, for example, *Salmonella typhi, Salmonella typhimurium; Yersinia* such as, for example, *Yersinia pestis, Yersinia pseudotuberculosis, Yersinia enterocolitica; Serratia marcescens; Aerobacter aerogenes; Enterobacter sakazakii; Acinetobacter,* such as, for example, *Acinetobacter baumannii, Acinetobacter beijerinckii, Acinetobacter bereziniae, Acinetobacter boissieri; Moraxella* such as, for example, *Moraxella catarrhalis* (synonym *Branhamella catarrhalis); Neisseria* such as, for example, *Neisseria meningitidis; Haemophilus* such as, for example, *Haemophilus influenzae; Pasteurella* such as, for example, *Pasteurella multocida; Pseudomonas* such as, for example, *Pseudomonas aeruginosa; Vibrio* such as, for example, *Vibrio cholerae, Vibrio fischeri, Stenotrophomonas maltophilia.*

More preferably, the Gram negative bacterium is selected from *Salmonella typhimurium* and *Escherichia coli.*

Within the scope of the invention, the Gram positive bacterium is preferably selected from: *Actinobacteria* such as, for example, *Tropheryma whipplei; Bacillus; Carnobacterium; Clostridium; Corynebacterium diphtheria; Enterococcus* such as, for example, *Enterococcus faecalis; Gardnerella vaginalis; Lactobacillus; Lactococcus; Listeria* such as, for example, *Listeria monocytogenes; Micrococcus; Staphylococcus* such as, for example, *Staphylococcus aureus; Streptococcus* such as, for example, *Streptococcus agalactiae, Streptococcus pneumoniae, Streptococcus pyogenes, Streptococcus viridans.* More preferably, the Gram positive bacterium is selected from *Listeria monocytogenes* and *Staphylococcus aureus.*

Within the scope of the invention, the fungus preferably is *Aspergillus brasiliensis* and the yeast preferably is *Candida albicans.*

Within the scope of the invention, the virus is preferably equipped with a capsid and an additional coating called pericapsid and has a genetic material consisting of DNA or RNA. Preferably, the virus is an adenovirus, papilloma virus (HPV), herpes virus, coronavirus, influenza virus, cytomegalovirus (CMV), HIV or Ebola virus.

A fourth object of the present invention is a pharmaceutical formulation comprising an antimicrobial peptide consisting of the amino acid sequence represented by the following general formula:

$$X_1LX_2WVX_3IWX_4X_5$$

wherein $X_1$, $X_2$, $X_3$, $X_4$ and $X_5$ are independently selected from K and R and wherein each amino acid is independently in the D or L configuration,

or a salt or a solvate thereof,

and at least one pharmaceutically acceptable excipient and/or carrier.

The pharmaceutical formulations according to the invention include, by way of example, those suitable for oral, parenteral (including subcutaneous, intradermal, intramuscular, intravenous and intra-articular) use, or for inhalation (including particularly small particle powders or mists that may be generated by means of various types of pressurized aerosol meters, nebulizers or insufflators). However, the most suitable route of administration may depend, for example, on the condition of the subject and the disease to be treated. The person skilled in the art is able to identify the route of administration which is most suited to the specific circumstances of the case.

The pharmaceutical formulations of the invention can be conveniently presented in unit dosage form and can be prepared by means of any one of the protocols well known in the pharmaceutical art. All the methods involve the step of combining the active ingredient (i.e., the peptide) with a carrier, including one or more pharmaceutically acceptable excipients. Generally, pharmaceutical formulations are prepared in a uniform way by combining the active ingredient with liquid carriers or finely divided solid carriers, or both, and then, if necessary, shaping the product into the desired formulation.

The pharmaceutical formulations of the present invention suitable for oral administration can be presented in the form of discrete units such as, for example, capsules, cachets or tablets, each containing a predetermined amount of the active ingredient; powder or granules; a solution or suspension in an aqueous liquid or in a non-aqueous liquid; or an oil-in-water liquid emulsion or a water-in-oil liquid emulsion. The active ingredient can also be presented as a bolus, electuary or paste. Several pharmaceutically acceptable excipients and carriers are described in standard formulation treaties, for example Remington's Pharmaceutical Sciences by E. W. Martin. See also Wang, Y.J. and Hanson, M. A., Journal of Parenteral Science and Technology, Technical Report No. 10, Supp.

Formulations for oral administration include, for example, suspensions which may contain, for example, microcrystalline cellulose for imparting bulkiness, alginic acid or sodium alginate as a suspending agent, methylcellulose as a viscosifier, and sweeteners or flavouring agents such as those already known in the pharmaceutical sector; immediate release tablets, which may contain, for example, microcrystalline cellulose, calcium phosphate, starch, magnesium stearate and/or lactose and/or other excipients, binders, extenders, disintegrants, diluents and lubricants such as those known in the pharmaceutical art.

Formulations for parenteral administration include, for example, sterile aqueous and non-aqueous solutions for injection which may contain antioxidants, buffers, bacteriostats and solutes which make the formulation isotonic; sterile aqueous and non-aqueous suspensions which may include surfactants and thickeners. The formulations can be presented in single-dose containers or in multi-dose containers, such as vials and sealed vials, and can be stored in lyophilized form, only requiring the addition of the sterile liquid carrier immediately prior to use.

Formulations for nasal aerosol or inhalation administration include, for example, saline solutions that may contain, for example, benzyl alcohol or other appropriate preservatives, which promote absorption to enhance bioavailability, and/or other solubilizing agents such as those known in the pharmaceutical art. In formulations for nasal aerosol or inhalation administration, the peptide of the invention is administered in the form of an aerosol from a pressurized container or a nebulizer, using a suitable propellant, for example, dichlorodifluoromethane, trichlorofluoromethane, dichlorotetrafluoroethane, carbon dioxide, or other suitable gas. In the case of a pressurized aerosol, the dosage unit can be determined by providing a valve to deliver a metered quantity. Preferred unit dosage formulations are those containing an effective dose, as previously described, or an appropriate ratio, of the active ingredient.

It should be emphasized that, in addition to the ingredients specifically mentioned above, the formulations of the present invention can include other conventional agents used in the pharmaceutical art, taking into account the type of formulation in question; for example, those suitable for oral administration may include flavouring agents.

The antimicrobial peptides of the invention are also appropriately administered as sustained and controlled release systems. Suitable examples of sustained release systems usable with the peptides of the invention include suitable polymeric materials, such as semi-permeable polymer matrices, for example films or microcapsules; specific hydrophobic materials, such as emulsions in suitable oils or ion exchange resins; and derivatives of the peptides of the invention, such as, for example, a salt or solvate.

A fifth object of the present invention is an antimicrobial composition comprising a carrier and an antimicrobial peptide consisting of the amino acid sequence represented by the following general formula:

$$X_1LX_2WVX_3IWX_4X_5$$

wherein $X_1$, $X_2$, $X_3$, $X_4$ and $X_5$ are independently selected from K and R and wherein each amino acid is independently in the D or L configuration,
or a salt or a solvate thereof.

According to one embodiment of the invention, the antimicrobial composition comprises at least one further antimicrobial peptide according to the invention and/or at least one further antimicrobial agent.
A sixth object of the present invention is the use of an antimicrobial peptide consisting of the amino acid sequence represented by the following general formula:

$$X_1LX_2WVX_3IWX_4X_5$$

wherein $X_1$, $X_2$, $X_3$, $X_4$ and $X_5$ are independently selected from K and R and wherein each amino acid is independently in the D or L configuration,
or a salt or a solvate thereof,
as an antimicrobial agent for the prevention of contamination of an article by and/or for the decontamination of an article (e.g., a food, a material, a container or a tool) from Gram negative bacteria, Gram positive bacteria, fungi, yeast and/or viruses.

A seventh object of the invention is an article bearing an antimicrobial peptide covalently bonded to a surface thereof, or bearing an antimicrobial peptide contained in a coating adhered to said surface, wherein said antimicrobial peptide consists of the amino acid sequence represented by the following general formula:

$$X_1LX_2WVX_3IWX_4X_5$$

wherein $X_1$, $X_2$, $X_3$, $X_4$ and $X_5$ are independently selected from K and R and wherein each amino acid is independently in the D or L configuration,
or a salt or a solvate thereof.

In the event that the peptide is covalently bonded to the surface of the article, the bonding occurs with reactive groups on the surface itself.
Preferably, the article is a container, a tool or a material for the storage or processing of food products, or a food.
According to a preferred embodiment, the antimicrobial peptide is contained in a coating applied to the surface of the article. In this case, the application provides that a liquid antimicrobial composition containing the peptide according to the invention is applied to the surface of said article and then left to dry. This composition can optionally comprise film-forming agents which form a film on the surface of the article and favour the permanence of the peptide thereon. Therefore, the aforementioned coating preferably comprises a film-forming polymer.
Alternatively, preferably, according to a different embodiment, the peptide is bonded with a covalent chemical bond to the surface of the article. In this case, the peptide is bonded by the formation of a covalent bond with reactive groups on the surface of the article. This makes it possible to obtain a surface characterized by a stable bactericidal activity for long periods of time.
Techniques for binding peptides or proteins to solid supports are known to those skilled in the art and vary depending on the material used.
For example, in the case of materials having metal (gold, silver, platinum) and semiconductor (titanium, zinc, tin, zirconium, germanium) surfaces, a silanization process can be used. This involves, for example, reacting the material to be treated with a mixture of sulfuric acid ($H_2SO_4$) and oxygen peroxide ($H_2O_2$), which are able to activate the aforementioned surfaces by creating bonds of surface atoms and hydroxyl groups (-OH) easily replaceable by more stable bonds such as Si-C or Au-S. The activated surfaces can covalently bind the peptides of the invention following treatment with a silanizing agent, such as aminopropyldimethylethoxysilane or aminopropyltriethoxysilane, and with a compound having two functional groups capable of forming the peptide covalent bond with the amino groups of the peptide, such as glutaraldehyde or bis-succinimide. These treatments are typical of the chemistry of aqueous solutions and for this reason they are referred to as wet processes, which are advantageous because they do not require particular technological equipment but only, preferably rigid, materials which can be wet and dried without difficulty.
In the case of plastic or polymeric surfaces, these can instead be functionalized to link the peptides of the invention either by applying the wet processes described above, or by applying high energy electromagnetic radiation (for example, laser, ultraviolet radiation, gamma rays). For the so-called soft materials such as non-rigid plastics, wet processes may not be completely effective, therefore the so-called dry functionalization processes based on the interaction of the surface with a gas plasma or electromagnetic radiation are preferred. The interaction of the surface of a polymer with electromagnetic radiation causes surface activation through the breaking of the accessible polymer bonds, therefore the C=C bonds become -C-C-, thereby allowing subsequent chemical modification of the surface itself. A similar operating principle applies to the other polymer surface activation method, which consists in the treatment thereof with ionized gas (gas plasma). This method is particularly advantageous since, as the plasma is cold, the temperature of the treated material does not reach high values with respect to room temperature. This method requires low pressure (0.1-100 Pa) and the presence of a working gas (usually $N_2$, $O_2$ or Ar, $CF_4$). [Hegemann, Dirk, Herwig Brunner, and Christian Oehr. "Plasma treatment of polymers for surface and adhesion improvement." Nuclear instruments and methods in physics research section B: Beam interactions with materials and atoms 208 (2003): 281-286].

Further antimicrobial applications of the antimicrobial peptides of the invention include, for example, the sanitization of plants or machinery intended for the processing of food products, or the use as a preservative in food products, for example for the prevention of contamination thereof by and/or the elimination of contamination thereof from *Listeria monocytogenes, Salmonella typhimurium, Escherichia coli, Staphilococcus aureus, Aspergillus brasiliensis* and *Candida albicans.* This use involves the use of the peptide on the surface and/or inside the food or on the surface of its packaging materials.

Preferably, the antimicrobial peptide is used as a preservative agent in food products. Alternatively, the peptide is used for application to the surface of food packaging materials, e.g., for the prevention of contamination by and/or the elimination of contamination from *Listeria monocytogenes, Salmonella typhimurium, Escherichia coli, Staphilococcus aureus, Aspergillus brasiliensis* and *Candida albicans* of said materials and of the foods contained therein.

## EXAMPLES

### Example 1 - Synthesis of the RiLK1 peptide

The peptide representative of the invention, RiLK1, having the sequence RLKWVRIWRR (SEQ ID NO: 1), was synthesized by solid phase peptide synthesis using the protective group Fluoromethoxycarbonyl (Fmoc).

Rink-Amide MBHA resin with a degree of substitution of 0.5 mmol/g was used as a solid support. The resin has a linker that provides an amide bond and releases the peptide amidated at the C-terminus.

At the end of the synthesis, the protective group was removed by treatment with a 40% (v/v) solution of piperidine in DMF, while the detachment from the resin and the removal of the protective groups from the amino acid side chains were obtained by treatment with an acidic solution consisting of 95% trifluoroacetic acid, 2.5% triisopropylsilane, and 2.5% $H_2O$ (v/v/v).

After the detachment from the solid support, the peptide was precipitated in cold ethyl ether, at -20°C. The sample was centrifuged at 3500 rpm for 5 minutes in order to collect the precipitate. The precipitate was dissolved in a mixture of $CH_3CN/H_2O$ (95:5), frozen and lyophilized.

This procedure can be used for the synthesis of all AMP peptides according to the present invention.

### Example 2 - Analysis of the solubility of the RiLK1 peptide in the D or L configuration

Within the scope of the definition of the chemical properties of the RiLK1 peptide in the L or D configuration, solubilization tests were carried out in different solvents and in a concentration range between 1 and 11 mg/ml, in order to identify the solubility limit, i.e., the maximum concentration of peptide dissolved in the solution. The tests were carried out in three of the most common solvents used in biological applications such as: ultrapure water; DPBS (Dulbecco's Phosphate Buffered Saline), i.e., a saline buffer containing potassium chloride (KCl), monobasic potassium phosphate ($KH_2PO_4$), sodium chloride (NaCl) and dibasic sodium phosphate ($Na_2HPO_4$-$7H_2O$) and finally in an organic solvent such as dimethylsulfoxide (DMSO). The data set out in Table 1 show that the RiLK1 peptide in either the L or the D configuration is able to completely dissolve in the various solvents examined up to a maximum concentration of 10 mg/ml.

The same tests carried out on the L and D configurations of the IDR-1018-K6 peptide (object of patent WO2019012158) showed that the L configuration has a much lower solubility limit in water and DPBS ($\leqq$5 mg/ml) compared to the peptide object of the present invention, RiLK1, whereas the D configuration is totally insoluble.

Table 1

| | Solvent | Outcome | Solubility limit |
|---|---|---|---|
| **RiLK1(L)** | Ultrapure water | Dissolved | 10 mg/ml |
| | 1X DPBS * (pH 7.1±0.1) | Dissolved | 10 mg/ml |
| | DMSO | Dissolved | 10 mg/ml |
| **RiLK1(D)** | Ultrapure water | Dissolved | 10 mg/ml |
| | 1X DPBS * (pH 7.1±0.1) | Dissolved | 10 mg/ml |
| | DMSO | Dissolved | 10 mg/ml |
| **IDR-1018-K6(L)** | Ultrapure water | Dissolved | 5 mg/ml |
| | 1X DPBS * (pH 7.1±0.1) | Dissolved | 5 mg/ml |
| | DMSO | Dissolved | 10 mg/ml |
| **IDR-1018-K6 (D)** | Ultrapure water | NOT dissolved | 0 |
| | 1X DPBS * (pH 7.1±0.1) | NOT dissolved | 0 |
| | DMSO | Dissolved | 10 mg/ml |

## Example 3 - Structural and conformational characterization of RiLK1

The structural and conformational characterization of the RiLK1 peptide was assessed by investigating its secondary structure by Circular Dichroism (CD) spectroscopy in the absence and in the presence of Sodium Dodecyl Sulfate (SDS) at a concentration of 3mM to mimic the condition of the bacterial cell membrane.

The CD assays were performed with a Jasco J-810 spectropolarimeter equipped with a thermostated cuvette holder. The samples were loaded into a 0.1 cm long quartz cuvette (Hellma Analytics) and the spectrum was analysed in the range from 190 nm to 250 nm at room temperature for each sample.

The CD spectra were acquired at a scan rate of 20 nm/min, and the recorded results represent an average of 5 scans.

The mean residue ellipticity ($[\theta]$, degrees $cm^2$ $dmol^{-1}$) was calculated using the following equation:

$$[\theta] = 100 \; \theta \, /cn1$$

wherein $\theta$ represents the ellipticity (m degrees), c is the concentration (mM) of the peptide, n is the number of residues.

The secondary structure was calculated through the DICHROWEB site (Whitmore L and Wallace BA, 2004, Nucleic Acids Research 32: 668-673, Whitmore L and Wallace BA, 2008, Biopolymers 89:392-400, Lobley et al, 2002, Bioinformatics 18:211-212), using three different algorithms (SELCON3, CONTIN-LL and CDSSTR) (Sreerema N and Woody RW, 1993, Analytical Biochemistry 287:252-260) and selecting the SMP180 protein as the comparison data set, which includes a high number of spectra of soluble and membrane proteins (Abdul-Gader A et al, 2011, Bioinformatics 27:1630-1636).

The secondary structure was also calculated through the BETSEL site (Micsonai A. et al, 2018, Nucleic Acids Res. 46: W315-W322; Micsonai A. et al, 2015, Proc Natl Acad Sci U S A; 112: E3095-E3103).

As shown in Figure 1, the CD spectrum of the RiLK1 peptide in water in the absence (solid black line) or in the presence (dashed black line) of SDS exhibits two negative bands at 200 nm and 215 nm, respectively. In the absence of the SDS solution, the peptide is unstructured; in the presence of this solution which mimics the presence of a bacterium, the peptide is structured and takes a mixed $\alpha$-helix/sheet conformation.

As shown in Figure 2, the CD spectrum of the RiLK1 peptide in sodium acetate buffer at pH 4 in the absence or presence of SDS still exhibits two negative bands. In the absence of the SDS solution, the peptide is unstructured (solid black line); in the presence of this solution, the peptide is structured (dashed black line) and takes a mixed $\alpha$-helix/$\beta$-sheet conformation.

As can be seen by comparing the spectra in Figure 1 with those in Figure 2, the conformation of the peptide and accordingly the profile of the spectra vary according to the environmental conditions, in this case according to the pH of the water which is different from the pH of the sodium acetate buffer.

The structural and conformational characterization of the RiLK1 peptide was assessed by investigating its secondary structure by $^1$H NMR spectroscopy in $H_2O$ and in a micellar solution of SDS at a concentration of 150mM to mimic the condition of the bacterial cell membrane.

The $^1$H NMR spectra in water and the intra-residue ($NH_i$-$\alpha CH_i$) and sequential ($\alpha CH_i$-$NH_{i+1}$) NOE signals revealed the presence of disordered isoenergetic structures (random coils) in rapid interconversion on the NMR time scale analysis, consistent with a random conformation of the peptide. In fact, the representative structures of the RiLK1 peptide in water show a random orientation of both polar and hydrophobic amino acid side chains.

This structure analysis was further validated by the lack of any long-range NOE signal. The RiLK1 peptide structures obtained by $^1$H NMR included in the 3 most populated clusters of molecules selected as representatives of the conformational state of the peptide are shown as cartoon models in Figure 3a (A, B).

The 1H NMR spectra in the presence of SDS revealed the induction of a specific iso-orientation of the positively charged amino acid side chains, with the formation of a central amphipathic $\alpha$-helix structure. The helical structure appears well defined and stabilized by hydrophobic interactions of amino acid residues located on the opposite face. It is important to note that, in this arrangement, all the charged side chains are iso-oriented and well prone to interact with the negatively charged surface of the SDS micelles. Furthermore, the two tryptophan residues within the RiLK1 sequence are located at an optimal distance for their mutual stacking, thus contributing to the stabilization of the helix. It should be pointed out that the 1H NMR spectra of the peptide IDR-1018-K6 (described in patent application WO2019012158) could not be acquired in the presence of micellar solutions of SDS since it is not soluble at the concentrations required to carry out these assays. In order to compare the two peptides, the structures were modelled as alpha-helices using specific software and the results obtained showed that the peptide IDR-1018-K6 of the prior art is not able to assume iso-oriented structures in the presence of micellar solutions of SDS due to its amino acid sequence and above all the lack of the two correctly spaced tryptophan residues. Therefore, it is possible to assert that the NMR analysis in the presence of SDS has shown that the RiLK1 peptide of the present invention is capable of adopting the helix conformation, whereas the IDR-1018-K6 peptide (object of patent WO2019012158) has poor solubility and a heterogeneous system not suitable for NMR structural characterization (Table 2).

The RiLK1 peptide structures obtained by $^1$H NMR included in the most populated cluster of molecules selected as representative of the conformational state of the peptide are shown as cartoon models in Figure 3b (A, B).

It should be noted that, in the experimental conditions used to acquire the NMR spectra, only the presence of the alpha-helix secondary structure elements adopted by the RiLK1 peptide could be assessed.

Table 2

| Peptide | H2O/D2O 90/10 v/v | H2O/SDS-d25 150 mM, pH 4.4 |
|---|---|---|
| RiLK1 | 1 mM | 0.9 mM |
| *structure* | random coil | central $\alpha$-helix |
| IDR-1018-K6 | 1 mM | insoluble |
| *structure* | random coil | ND |

## Example 4 - Kinetics of RiLK1 structural rearrangements

The kinetics of the structural rearrangements of the RiLK1 peptide were assessed over 24 hours by Circular Dichroism (CD) spectroscopy and fluorescence spectroscopy.

0.1 g/L of RiLK1 peptide was dissolved in 10 mM sodium acetate buffer at pH 4, in the absence or presence of 3mM SDS.

As shown in Figure 4, the CD spectrum of the RiLK1 peptide in the presence of SDS maintains in the 24-hour period a negative band between 210-215 nm (lines 2-8), i.e., the peptide remains structured for 24 hours and maintains the mixed $\alpha$-helix/sheet conformation.

As shown in Figure 5, the fluorescence spectrum of the RiLK1 peptide in the presence of SDS in the 24-hour period maintains a lower wavelength peak, between 320-340 nm (lines 2-8), compared to the wavelength of the peak in the absence of SDS, which is between 340-360 nm (line 1). The fluorescence spectrum confirms that the peptide in the presence of 3 mM SDS in the 24-hour period undergoes conformational rearrangements following the interaction of tryptophan with the neighbouring amino acids, without disrupting the secondary structure elements, as shown in the same analysis performed by circular dichroism (Figure 4).

The data reported in Table 3 below, obtained by *in silico* analysis of the structural rearrangement kinetics of the RiLK1 peptide in the 24-hour period using two different servers, BESTSEL and Dichroweb, show that, following the addition of the 3 mM SDS solution, a predominance of the $\beta$-sheet secondary structure element over the $\alpha$-helix is observed in both servers (53.6% vs 8.4% in BESTSEL; 47% vs 29% in DICHROWEB).

Table 3

| RiLK1 | | | | | | |
|---|---|---|---|---|---|---|
| | Bestsel | | | DichroWeb CONTIN-LL | | |
| | α-helix | β-sheet | Random | α-helix | β-sheet | Random |
| w/o 3mM SDS | 1.2% | 47.3% | 51.5% | 4% | 35% | 61% |
| t=0 w/ 3mM SDS | 7.4% | 51.4% | 41.3% | 22% | 38% | 40% |
| t= 5min w/ 3mM SDS | 4.4% | 53.4% | 42.2% | 22% | 42% | 36% |
| t= 30min w/ 3mM SDS | 11% | 48.7% | 40.3% | 25% | 38% | 37% |
| t= 1h w/ 3mM SDS | 9.8% | 51.1% | 39.1% | 23% | 38% | 39% |
| t= 5h w/ 3mM SDS | 6.7% | 54% | 39.3% | 19% | 43% | 38% |
| t= 6h w/ 3mM SDS | 8% | 52% | 40.1% | 19% | 44% | 37% |
| t= 24h w/ 3mM SDS | 8.4% | 53.6% | 38% | 29% | 47% | 24% |

Table 4 below shows the data obtained by *in silico* analysis of the structural rearrangement kinetics of the RiLK1 peptide in the 24-hour period using the Dichroweb server; these data show that, following the addition of the SDS solution, the RiLK1 peptide after 24 hours shows a 47% predominance of the β-sheet secondary structure element. Furthermore, the RiLK1 peptide after 24 hours shows a low random portion, ranging from 61% in the absence of SDS to 24% in the presence of 3 mM SDS after 24 hours.

Table 4

| RiLK1 | | | |
|---|---|---|---|
| | DichroWeb CONTIN-LL | | |
| | α-helix | β-sheet | Random |
| w/o 3mM SDS | 4% | 35% | 61% |
| t=0 w/ 3mM SDS | 22% | 38% | 40% |
| t=5 min w/ 3mM SDS | 22% | 42% | 36% |
| t=30 min w/ 3mM SDS | 25% | 38% | 37% |
| t=60 min w/ 3mM SDS | 23% | 38% | 39% |
| t=300 min w/ 3mM SDS | 19% | 43% | 38% |
| t=360 min w/ 3mM SDS | 19% | 44% | 37% |
| t=24 hours w/ 3mM SDS | 29% | 47% | 24% |

### Example 5 - Analysis of the thermal structural stability of RiLK1

0.1 g/L of RiLK1 peptide was dissolved in 10mM Tris HCL at pH 7 and 3mM SDS. The prepared samples were incubated for 24 hours at 4°C or 25°C and analysed at time zero and after 24 hours.

As shown in Figure 6, the CD spectrum of the RiLK1 peptide in the presence of 3 mM SDS maintains a negative band between 210-215 nm in the 24-hour period both at the temperature of 4°C (line 2) and at the temperature of 25°C (line 3), i.e., the peptide remains structured for 24 hours at these temperatures and maintains the mixed α-helix/sheet conformation.

The data reported in Table 5 below, obtained by *in silico* analysis of the structural rearrangement kinetics of the RiLK1 peptide in the 24-hour period using two different servers, BESTSEL and Dichroweb, show that RiLK1 is remarkably stable under the different environmental conditions investigated, with a predominance of the β-sheet over the α-helix.

Table 5

| RiLK1 | | | | | | |
|---|---|---|---|---|---|---|
| | Bestsel | | | DichroWeb CONTIN-LL | | |
| | $\alpha$-helix | $\beta$-sheet | Random | $\alpha$-helix | $\beta$-sheet | Random |
| t=0 | 17.9% | 37.1% | 44.9% | 21% | 43% | 36% |
| 4°C; t=24 hours | 6.5% | 46% | 47.5% | 25% | 37% | 38% |
| 25°C; t=24 hours | 6.8% | 49.7% | 43.6% | 24% | 39% | 37% |

On the other hand, Table 6 below shows the data obtained by *in silico* analysis of the structural rearrangement kinetics of the RiLK1 peptide in the 24-hour period using the Dichroweb server; these data show that the RiLK1 peptide after 24 hours shows a predominance of the $\beta$-sheet secondary structure element even at 90°C.

Table 6

| RiLK1 | | | |
|---|---|---|---|
| | CONTIN-LL | | |
| | $\alpha$-helix | $\beta$-sheet | Random |
| t=0 | 21% | 43% | 36% |
| 4°C; t=24h | 25% | 37% | 38% |
| 25°C; t=24h | 24% | 39% | 37% |

Taken together, these data show that RiLK1 maintains its structure almost unchanged in the range from 4°C to 25°C and is therefore characterized by good thermal stability.

## Example 6 - Analysis of the pH structural stability of RiLK1

0.1 g/L of RiLK1 peptide was dissolved in 10 mM concentration solutions at different pHs, in particular: glycine-HCL buffer (pH 2); sodium acetate buffer (pH 4); tris-HCL buffer (pH 7); glycine-NaOH buffer (pH 9). After a 1-hour incubation at 25°C, samples of each solution containing 3mM SDS were prepared. The results obtained are reported in Figure 7. This figure shows that the CD spectrum of the RiLK1 peptide obtained by monitoring the sample for 24 hours (solid black line at time point = 0; dashed black line at time point = 24 hours) in the presence of SDS maintains a negative band between 210-215 nm in the 24-hour period under the different pH conditions tested, namely pH 2 (A); pH 4 (B); pH 7 (C); pH 9 (D), i.e., the peptide remains structured and maintains the mixed $\alpha$-helix/sheet conformation in the 24-hour period under these pH conditions.

The data reported in Table 6 below, obtained by *in silico* analysis of the structural rearrangement kinetics of the RiLK1 peptide in the 24-hour period under the different pH conditions tested and using two different servers, BESTSEL and Dichroweb, show that RiLK1 is remarkably stable under the different pH conditions investigated, with a predominance of the $\beta$-sheet and a decrease in the $\alpha$-helix component.

Table 7

| RiLK1 | | | | | | |
|---|---|---|---|---|---|---|
| | Bestsel | | | DichroWeb CONTIN-LL | | |
| | $\alpha$-helix | $\beta$-sheet | Random | $\alpha$-helix | $\beta$-sheet | Random |
| pH2; t=0 | 18.5 | 37 | 44.4 | 22% | 39% | 39% |
| pH2; t=24 hours | 13.2 | 42.5 | 44.3 | 17% | 44% | 39% |
| pH4; t=0 | 7.7 | 39.8 | 52.4 | 23% | 41% | 36% |
| pH4; t=24 hours | 13.3 | 45.5 | 41.2 | 24% | 41% | 35% |
| pH7; t=0 | 15 | 39.5 | 45.4 | 25% | 39% | 36% |
| pH7; t=24 hours | 15.5 | 43.8 | 40.8 | 19% | 44% | 37% |

(continued)

| RiLK1 | | | | | | |
|---|---|---|---|---|---|---|
| | Bestsel | | | DichroWeb CONTIN-LL | | |
| | $\alpha$-helix | $\beta$-sheet | Random | $\alpha$-helix | $\beta$-sheet | Random |
| pH 9; t=0 | 13.7 | 42 | 44.4 | 19% | 42% | 39% |
| pH 9; t=24 hours | 13.4 | 43.8 | 42.9 | 29% | 47% | 24% |

Table 8 below shows the data obtained by *in silico* analysis of the structural rearrangement kinetics of the RiLK1 peptide in the 24-hour period using the Dichroweb server; these data show that the RiLK1 peptide after a 24-hour incubation at the different pHs tested and in the presence of 3 mM SDS maintains a predominance of the $\beta$-sheet secondary structure element over the $\alpha$-helix.

Table 8

| RiLK1 | | | |
|---|---|---|---|
| | CONTIN-LL | | |
| | $\alpha$-helix | $\beta$-sheet | Random |
| pH 2 | 22% | 39% | 39% |
| pH 4 | 23% | 41% | 36% |
| pH 7 | 25% | 39% | 36% |
| pH 9 | 19% | 42% | 39% |

## Example 7 - Analysis of the pH and thermal structural stability of RiLK1 in a micellar solution

The RiLK1 peptide (at a concentration of 50 $\mu$M) was dissolved in 10 mM buffer solutions at two different pHs, in particular: glycine-HCL buffer (pH 2) and glycine-NaOH buffer (pH 9). After a 1-hour incubation at 25°C, samples of each solution in the presence of 150 mM SDS were prepared and incubated for 24 hours at 25°C.

As shown in Figure 8 A-B, the fluorescence spectra of the RiLK1 peptide in the 24-hour period remain almost unchanged at the two pH conditions tested, indicating a remarkable stability of the peptide, with slight differences in the maximum fluorescence intensity due to the different pH-induced conformational rearrangement of the peptide, which results in a greater or lesser solvent exposure of the tryptophan residues.

Furthermore, the RiLK1 peptide was dissolved in water at a concentration of 50 $\mu$M in the presence of a micellar solution of 150 mM SDS. The prepared samples were incubated for 24 hours at 4°C or 25°C and analysed by fluorescence spectroscopy. As shown in Figure 8 C-D, the fluorescence spectra are superimposable in the 24-h period, demonstrating that RiLK1 is also characterized by high structural stability at 4°C and 25°C in a micellar solution of SDS.

## Example 8 - Stability analysis of RiLK1 in conditions of use

The stability analysis of the RiLK1 peptide in the presence of a high concentration saline solution (1M NaCl) was carried out by reverse phase (RP) HPLC chromatography, performed on a $\mu$Bondpack C18 column. 50$\mu$M RiLK1 peptide was dissolved in 1 M NaCl and the samples were incubated at 25°C and monitored for 9 days.

As shown in Figure 9, all the chromatographic plots obtained, i.e., at time point = 0; time point = 5 hours; time point = 24 hours; time point = 29 hours; time point = 48 hours; time point = 53 hours; time point = 6 days; time point = 9 days, are practically superimposable, demonstrating that neither precipitation nor degradation phenomena occur during the incubation of the RiLK1 peptide, and that the peptide is extremely stable under these conditions up to a maximum of 9 days. Therefore, RiLK1, the object of this invention, has characteristics and structural properties such as to be used in the food industry in brine-based preparations, typical of dairy products such as mozzarella.

The data reported in Table 9 below confirm the stability of the RiLK1 peptide up to 9 days.

Table 9

| 50 $\mu$M RiLK1/ 1M NaCl | |
|---|---|
| time point | yield |
| 0 | - |
| 5 hours | +6% |
| 24 hours | +6% |
| 29 hours | +3% |
| 48 hours | -1.5% |
| 53 hours | +4.3% |
| 6 days | -6.3% |
| 9 days | -8% |

### Example 9 - Analysis of the bactericidal activity of RiLK1

### Example 9a - Assessment of the concentration capable of 50% bacterial growth inhibition (IC$_{50}$)

The bactericidal activity of the RiLK1 peptide was assessed against both Gram positive *(Listeria monocytogenes LM2* and *Staphilococcus aureus)* and Gram negative *(Salmonella typhimurium* and *Escherichia coli)* pathogenic bacteria.

For the four bacterial species selected, certified and characterized strains, as indicated in the following Tables 10 and 11, were used.

The assessment of the concentration of the two peptides capable of inhibiting 50% of bacterial growth (IC$_{50}$) was performed by the broth microdilution assay as described in Wang HX and Ng TB (2003, Peptides 24:969-972).

The standard deviations and the IC50s for triplicate incubations of each plate were determined using GraphPad Prism version 6.00 (Graph-Pad Software, La Jolla California USA).

*Staphylococcus aureus*

A control stock suspension was prepared in which $10^3$ CFUs of S. *aureus* were inoculated in 10 ml of BPW. 5 mM stock solutions of the two peptides were then prepared and serial dilutions (100 to 1$\mu$M) were carried out in BPW and inoculated with $10^3$ CFUs of S. *aureus,* and then incubated for 6 hours at 37°C. At the same time, control samples were prepared and treated in the same way but without the addition of the two peptides.

50 $\mu$l of each bacterial suspension were poured onto blood agar or rabbit plasma fibrinogen agar Petri dishes and incubated for 20 hours at 37°C.

All experimental conditions investigated used the plate count method to estimate the bactericidal activity of the peptides. Specifically, the numbers of colonies grown on agar plates seeded with the bacterial suspensions in the absence or presence of the individual peptide dilutions were counted and compared. The standard deviations of the triplicate incubations of each plate were determined using statistical software.

*Listeria monocytogenes*

A control stock suspension was prepared in which $10^3$ CFUs of *L. monocytogenes* were inoculated in 10 ml of Half Fraser Broth and serial dilutions (100 to 0.01$\mu$M) of the suspension were performed. 5 mM stock solutions of the two peptides were then prepared in DMSO and serial dilutions (100 to 0.01$\mu$M) were carried out in Fraser Broth and inoculated with $10^3$ CFUs (colony forming units) of *L. monocytogenes,* and then incubated for 6 hours at 37°C. At the same time, control samples were prepared and treated in the same way but without the addition of the two peptides. 50 $\mu$l of each bacterial suspension were seeded on different culture plates: blood agar and ALOA (Oxoid, Basingstoke, UK), which were then incubated for 24-48 hours at 37°C. Each dilution series included control plates with bacteria only and DMSO without the peptide.

*Salmonella typhimurium*

A control stock suspension was prepared in which $10^3$ CFUs of S. *typhimurium* were inoculated in 10 ml of BPW (Oxoid, Basingstoke, UK). 5mM stock solutions of the two peptides were then prepared and serial dilutions (100 to 1$\mu$M)

were carried out in BPW and inoculated with $10^3$ CFUs of *S. typhimurium,* and then incubated for 6 hours at 37°C. 50 μl of each bacterial suspension were seeded on Petri dishes with blood agar or chromogenic agar (Oxoid, Basingstoke, UK) and incubated for 20 hours at 37°C. Each dilution series included control plates inoculated with DMSO without the peptide and control plates with bacteria only.

*Escherichia coli*

A control stock suspension was prepared in which $10^3$ CFUs of *E. coli* were inoculated in 10 ml of BPW (Oxoid, Basingstoke, UK). 5mM stock solutions of the two peptides were then prepared and serial dilutions (100 to 1μM) were carried out in BPW and inoculated with $10^3$ CFUs of *E. coli,* and then incubated for 6 hours at 37°C. 50 μl of each bacterial suspension were seeded on Petri dishes with blood agar or chromogenic agar (Oxoid, Basingstoke, UK) and incubated for 20 hours at 37°C. Each dilution series included control plates inoculated with DMSO without the peptide and control plates with bacteria only.

Figure 10 depicts the dose-response curve obtained with the RiLK1 peptide against S. *aureus* (A)*, L. monocytogenes* (B), *S. thyphimurium* (C)*,* and *Escherichia coli* (D)*.*

On the basis of the dose-response curves obtained, the $IC_{50}$ values (peptide concentration capable of inhibiting 50% of bacterial growth) against the bacterial strains mentioned above were determined.

The data reported in Table 10 show that the RiLK1 peptide exhibits strong bactericidal activity against all the tested bacteria ($IC_{50} < 2$ μM); in particular, a stronger bactericidal activity is observed against *S. thyphimurium, E. coli* and *L. monocytogenes* ($IC_{50} < 1.5$ μM) and an even stronger bactericidal activity is observed against *L. monocytogenes* ($IC_{50}$ 0.46 μM).

Table 10

| Bacterium | RiLK1 $IC_{50}$ [μM] |
|---|---|
| *S. aureus* | 1.98 |
| *L. monocytogenes (LM2)* | 0.46 |
| *S. typhimurium* | 1.30 |
| *E. coli* | 1.20 |

## Example 9b - Evaluation of the Minimum Bactericidal Concentration (MBC)

The evaluation of the Minimum Bactericidal Concentration (MBC), i.e., the lowest concentration of antimicrobial agent capable of 99.9% inhibition of bacterial growth on plates, was carried out as described in Bilikova et al, (2015, Peptides 68:190-196).

The data reported in the following Table 11 show that the RiLK1 peptide exhibits strong bactericidal activity against all the bacteria tested (MBC <20 μM); in particular, high bactericidal activity is observed against *L. monocytogenes, S. thyphimurium* and *E. coli* (MBC <5 μM), which in the case of *L. monocytogenes* and *E. coli* even reaches a value of MBC 2μM. It should be noted that the peptide object of the present invention is more powerful and efficient than the peptide IDR-1018-K6 (described in patent application WO2019012158) against the pathogenic bacteria *L. monocytogenes* and S. *typhimurium,* with bactericidal concentration values 4-fold and 7-fold lower, respectively, compared to those shown by IDR-1018-K6.

Table 11

| Bacterium | RiLK1 MBC [μM] | IDR-1018-K6 MBC [μM] |
|---|---|---|
| *S. aureus* | 16 | 15 |
| *L. monocytogenes (LM2)* | 2 | 8 |
| *S. typhimurium* | 4 | 25 |
| *E. coli* | 2 | 2 |

The images in Figure 11a represent photographic images of (1) the control plate incubated with *S. aureus* without the addition of the RiLK1 peptide, (2) the plate incubated with *S. aureus* and treated with 2μM RiLK1 peptide, (3) the plate incubated with *S. aureus* and treated with 16 μM RiLK1 peptide, which shows that at a concentration of 16 μM this peptide is able to inhibit almost 100% of the bacterial growth.

The images in Figure 11b represent photographic images of (1) the control plate incubated with *L. monocytogenes* without the addition of the RiLK1 peptide, (2) the plate incubated with *L. monocytogenes* and treated with 0.8 $\mu$M RiLK1 peptide, (3) the plate incubated with *L. monocytogenes* and treated with 2 $\mu$M RiLK1 peptide, which shows that at a concentration of 2 $\mu$M this peptide is able to inhibit almost 100% of the bacterial growth.

The images in Figure 11c represent photographic images of (1) the control plate incubated with *S. typhymurium* without the addition of the RiLK1 peptide, (2) the plate incubated with *S. typhymurium* and treated with 0.8 $\mu$M RiLK1 peptide, (3) the plate incubated with *L. monocytogenes* and treated with 2 $\mu$M RiLK1 peptide, which shows that at a concentration of 2 $\mu$M this peptide is able to inhibit almost 100% of the bacterial growth.

The images in Figure 11d represent photographic images of (1) the control plate incubated with *E. coli* without the addition of the RiLK1 peptide, (2) the plate incubated with *E. coli* and treated with 0.8 $\mu$M RiLK1 peptide, (3) the plate incubated with *E. coli* and treated with 2 $\mu$M RiLK1 peptide, which shows that at a concentration of 2 $\mu$M this peptide is able to inhibit almost 100% of the bacterial growth.

### Example 10 - Analysis of the cytotoxicity against human cell lines

The possible cytotoxicity of the RiLK1 peptide for eukaryotic cells was tested by performing cell growth and count experiments using three different human cell lines: HaCat (Primary Epidermal Keratinocytes), WI-38 (Diploid human cell line composed of fibroblasts derived from lung tissue), and TIG-3-20 (Fibroblast cell line derived from Japanese fetal lung).

Experiments were carried out in the absence or presence of the peptide at different concentrations: 1 $\mu$M, 2.5 $\mu$M, 5 $\mu$M, 10 $\mu$M.

As shown in the images of Figure 12, all human cell line samples tested after 24 hours of incubation in the presence of RiLK1 at the different concentrations (RiLK1_1, RiLK1_2.5, RiLK1_5 and RiLK1_10) show no signs of morphological changes nor a change in the cell number compared to the untreated cell sample (Ctrl).

### Example 11 - Analysis of the activity against fungi and yeasts

### Example 11a - Evaluation of the RiLK1 peptide concentration capable of inhibiting the growth of the fungus *Aspergillus brasiliensis*

The antifungal activity of the RiLK1 peptide was determined against *Aspergillus brasiliensis*. For this purpose, a stock culture was prepared in which $10^5$ CFUs of *A. brasiliensis* were inoculated in 10 ml of buffered peptone water. A reference strain (ATCC 9341) was used for this fungal species. The culture was incubated for 6 h at 37°C with the RiLK1 peptide at 25 $\mu$M concentrations.

At the same time, control samples were incubated without adding the peptide. 100 $\mu$l of the samples thus prepared were seeded on DG18 plates (Dichloran 18% Glycerol Agar - ISO 21527-2) and incubated at 25°C for 7 days.

All experimental conditions investigated used the plate count method to estimate the fungicidal activity of the peptide. Specifically, the numbers of colonies grown on agar plates seeded with the fungal suspensions in the absence or presence of the individual peptide dilutions were counted and compared. The standard deviations of the triplicate incubations of each plate were determined using statistical software. The evaluation of the Minimum Fungicidal Concentration (MFC), i.e., the lowest concentration of antifungal agent capable of 99.9% inhibition of fungal growth on plates, was carried out as described in Bilikova et al, (2015, Peptides 68:190-196).

As shown in Figure 13a, the RiLK1 peptide has strong fungicidal activity against the tested fungus, with an MFC value less than or equal to 25 $\mu$M. It should be noted that the peptide IDR-1018-K6 (object of patent WO2019012158) against the same fungus and under the same experimental conditions is not active, with a post-treatment decrease in the fungal load of 0 Log compared to the significant decrease induced by RiLK1 (5 Log reduction).

### Example 11b - Evaluation of the RiLK1 peptide concentration capable of inhibiting the growth of the yeast *Candida albicans*

The inhibitory activity of the RiLK1 peptide was determined against the yeast *Candida albicans*. A stock culture was prepared in which $10^5$ CFUs of *C. albicans* were inoculated in 10 ml of buffered peptone water. A reference strain (ATCC 14053) was used for this species. The culture was incubated for 6 h at 37°C with the RiLK1 peptide at 25 $\mu$M concentrations. At the same time, control samples were incubated without adding the peptide. 100 $\mu$l of the samples thus prepared were seeded on DG18 plates (Dichloran 18% Glycerol Agar - ISO 21527-2) and incubated at 25°C for 7 days.

All experimental conditions investigated used the plate count method to estimate the antimycotic activity of the peptide. Specifically, the numbers of colonies grown on agar plates seeded with the cultures in the absence or presence of the individual peptide dilutions were counted and compared. The standard deviations of the triplicate incubations of each plate were determined using statistical software. The evaluation of the Minimum Fungicidal Concentration (MFC), i.e., the lowest concentration of antifungal agent capable of 99.9% inhibition of fungal growth on plates, was carried out as described in Bílikova et al, (2015, Peptides 68:190-196).

As shown in Figure 13b, the RiLK1 peptide has strong antimycotic activity against C. *albicans,* with an MFC value less than or equal to 25 μM. It should be noted that, as shown in Table 12, the peptide IDR-1018-K6 (object of patent WO2019012158) against the same fungus and under the same experimental conditions exhibits low efficiency, with a post-treatment decrease in the fungal load of 1.0 Log compared to the significant decrease induced by RiLK1 (5 Log reduction).

Table 12

| Fungus | RiLK1 Drop Log CFU/ml | IDR-1018-K6 Drop Log CFU/ml |
|---|---|---|
| *A. brasiliensis* | 5 | 0 |
| *C. albicans* | 5 | 1.0 |

## Example 12: Effects of the treatment with RilK1-functionalized PP discs on mozzarella

### Example 12a: Activation and functionalization of polymeric surfaces with the RiLK1 peptide

An 800 μm-thick polypropylene (PP) solid support was first subjected to corona treatment, a technique that has the function of enhancing the adhesion and wettability of non-polar materials such as plastics consisting of long polymer chains, through the creation of reactive chemical groups on the polymeric surface, which then favour its functionalization with bioactive molecules such as RiLK1.

Once the activation was completed on both faces, 5 cm-diameter PP discs were incubated for 18h at 25°C in 5 ml of a 50 μM dimethylsulfoxide (DMSO) solution of the RiLK1 peptide in the presence of approximately 10 per ml solution of molecular sieves with 4 Å-diameter pores, i.e., crystalline aluminosilicates capable of adsorbing the water molecules produced during the formation of the peptide bond.

### Example 12b: Yield of immobilization and release

At the end of the functionalization reaction, the incubated solution was collected and analysed by reverse phase high performance liquid chromatography (RP-HPLC). This technique allowed the peptide bond yield to be assessed by determining the amount of unreacted RiLK1 vs that initially contacted with the polymeric surface.

As shown in Figure 14, the immobilization efficiency was found to be 91.5%, a value calculated by measuring the ratio between the peptide peak areas at time point t = 18h and time point t = 0.

The testing of the release of the RiLK1 peptide from the functionalized polymeric surface was carried out by immersing the same in 5 mL of $H_2O$ for 24h at a temperature of 25°C. At the end of the incubation, the liquid was recovered, concentrated and analysed by RP-HPLC chromatography to quantify the amount of release.

This analysis showed high stability in an aqueous solution of the functionalized polymeric system, as no release of the peptide was shown after 24h of incubation.

### Example 12c: Effects of the treatment with RilK1-functionalized PP discs on the shelf life of mozzarella

The RiLK1-functionalized PP discs (PPs) were used to carry out tests on the shelf-life of buffalo mozzarella, which is extremely reduced particularly when it is produced with raw or thermised milk, due to chemical-physical and biochemical alterations induced by spoilage microorganisms and enzymatic activities. At the same time, the possibility of obtaining buffalo milk derivatives from non-thermally "stressed" milk allows the so-called non-starter flora, which gives the food peculiarity and typicality characteristics, to be safeguarded. Normally, the shelf life of a so-called "over the counter" type of product is 4/5 days. 50 g morsels of mozzarella weighed in sterile conditions, which were then stored in the presence of functionalized and non-functionalized PPs, were used for the analytical protocols. Specifically, the preparation of the samples was as follows: in round-bottom containers, the diameter of which is equal to that of the disc, containing 200 ml of preserving liquid and a morsel of mozzarella, two PP discs were placed, one below and the other above the food matrix, sealed with parafilm and stored at a refrigeration temperature of 4°C until the expiry of the agreed times (5, 8 and 10 days). For the microbiological assays, 10 g of sample were weighed at the different time points, placed in a sterile bag equipped with a filter, diluted with peptone water (Buffered Peptone Water) in a 1:10 ratio and homogenized by Stomacher; 1 ml of the sample thus obtained was placed on a plate and counted according to the ISO 4833-1 methods for the TBL and the ISO 21527-1 methods for yeasts. As can be seen from the data reported in Tables 13 and 14, mozzarella stored for 10 days in the presence of PPs binding the RiLK1 peptide shows a 52% inhibition of the Total Bacterial Load and an 80.4% inhibition of yeasts when compared with mozzarella stored for the same number of days in the presence of non-functionalized PPs. This condition results in a 5-day increase in shelf life, which is a significant range in business dynamics concerning the marketing of an over-the-counter product.

Table 13

| TBL (Total mesophilic Bacterial Load) | |
| --- | --- |
| Functionalized PP disc-mozzarella contact times | % TBL inhibition compared to control |
| 5 d | 82% |
| 8 d | 50% |
| 10 d | 52% |

Table 14

| Yeasts | % inhibition |
| --- | --- |
| 5 d | 46.7% |
| 8 d | 80% |
| 10 d | 80.4% |

**Example 13**: **Analysis of the bactericidal activity of further AMP peptides according to the invention**

The bactericidal activity of further AMP peptides according to the invention, in particular of the RiLK31 peptide (RLRWVKIWKK, SEQ ID NO:31) and the RiLK3 peptide (RLRWVRIWRR, SEQ ID NO:3), was tested. The bactericidal activity was expressed as a percentage of viable cells in terms of CFU (Colony Forming Units) in a bacterial sample exposed to each peptide compared to those in a control bacterial sample in the absence of the peptide. All tests were carried out with the same peptide concentration, corresponding to 15 $\mu$M. The results obtained are shown in Table 15.

Table 15

| Peptide | E. coli | Staphylococcus | Salmonella | Listeria |
| --- | --- | --- | --- | --- |
| RiLK1 | 100% | 96.9% | 100% | 99.5% |
| RiLK31 | 100% | 83% | 100% | 98.8% |
| RiLK3 | 100% | 96% | 100% | 99% |

Table 15 shows that the further tested AMP peptides have a bactericidal activity comparable to that of the RiLK1 peptide used as the representative peptide in experiments 1-12 of the present description.

**Example 14: Characterization of further AMP peptides of the invention by computational analysis and comparison with the HE10 peptide of the prior art**

In bioinformatics, sequence alignment is a way to organize DNA, RNA or protein sequences in order to identify similarity regions which may result from functional, structural or evolutionary relationships between the sequences. Aligned sequences of amino acid residues are typically represented as rows in a matrix [Analysis Tool Web Services from the EMBL-EBI. (2013) McWilliam H, Li W, Uludag M, Squizzato S, Park YM, Buso N, Cowley AP, Lopez R. Nucleic acids research 2013 Jul;41(Web Server issue):W597-600 doi:10.1093/nar/gkt376; Principles and Methods of Sequence Analysis - Sequence - Evolution - Function - NCBI Bookshelf (Chapter 4) Koonin EV, Galperin MY. Sequence - Evolution - Function: Computational Approaches in Comparative Genomics. Boston: Kluwer Academic; 2003; Hans G. Boman (1995). Peptide antibiotics and their role in innate immunity. Annu. Rev. Immunol. 1995. 13:61-92]. Multiple sequence alignment highlights similarity areas, which may be associated with specific features, i.e., structural/functional biases that can be more conserved than other regions.

In protein sequence alignments, the degree of similarity between amino acids occupying a particular position in the sequence can be interpreted as an approximate measure of a particular conserved region or motif in the sequence. The absence of substitutions, or the presence of highly conservative substitutions alone (i.e., the substitution of amino acids whose side chains have similar biochemical properties) in a particular region of the sequence suggests that this region may have structural or functional importance [Hans G. Boman (1995). Peptide antibiotics and their role in innate immunity. Annu. Rev. Immunol. 1995. 13:61-92; Markéta Pazderková, Petr Malo, Vlastimil Zíma, Katerina Hofbauerová, Vladimír Kopecký Jr., Eva Kocišová, Tomáš Pazderka, Václav Cerovský and Lucie Bednárová (2019). Interaction of Halictine-Related Antimicrobial Peptides with Membrane Models. Int. J. Mol. Sci., 20, 631; doi:10.3390/ijms20030631; Igor Zelezetsky, Alessandro Tossi (2006). Alpha-helical antimicrobial peptides-Using a sequence template to guide structure-activity relationship studies. Biochimica et Biophysica Acta 1758: 1436-1449; Yang Wang, Jianbo Chen, Xin Zheng, Xiaoli Yang, Panpan M, Ying Cai, Bangzhi Zhang and Yuan Chena (2014). Design of novel analogues of short antimicrobial peptide anoplin with improved antimicrobial activity. J. Pept. Sci.; 20: 945-951. DOI 10.1002/psc.2705].

The presence of the same amino acid residues but in different positions in two amino acid sequences is not a gain but on the contrary leads to a decrease in the similarity score between the two peptides, as it can cause a profound structural and functional alteration.

When comparing two peptides, the correct way to identify variations and define similarity is to align the two sequences. This means placing one sequence on top of the other, so that it is easier to highlight which positions are the same and which are different. After performing the alignment, two quantitative parameters can be extracted from each pairwise comparison, namely identity and similarity. Identity defines the percentage of directly matching amino acids in the alignment. Similarity occurs when an amino acid is replaced with a similar residue, so that the physical-chemical properties are retained. For example, a change from arginine to lysine maintains the +1 positive charge. This change is much more likely to be acceptable, as the two residues have similar properties and do not impair the function of the peptide. Therefore, the percentage similarity of two sequences is the sum of the identical matches plus the similar ones. The similarity degree depends on the criteria that are used to compare two amino acid residues.

For example, the claimed peptide RiLK1 (RLKWVRIWRR, SEQ ID NO:1) and the prior art HE10 peptide (VRLIVRIWRR, SEQ ID NO: 33) differ from one another in four positions out of a total of 10 positions. Accordingly, they are 60% identical. However, considering the four substitution pairs, R > V, L > R, K > L, W > I, it appears that the biochemical properties and the size of the residues in each pair are significantly different. Therefore, as illustrated below, the substitution of the first four residues in the claimed peptide RiLK1 compared to HE10 significantly modifies the physical-chemical and structural properties of the peptide.

<u>RLKW</u>VRIWRR          Peptide - RiLK1 (SEQ ID NO:1)

<u>VRLI</u>VRIWRR          Peptide HE10 (SEQ ID NO: 33)

The underlined residues correspond to the positions where the two peptides differ.

As clearly shown in Table 16, the hydrophobicity, hydropathicity, amphipathicity, hydrophilicity, net charge and propensity for a disordered conformation of the claimed RiLK1 peptide are significantly different from those of HE10. These properties are known to strongly affect the characteristics of the peptides and modulate their physical-chemical characteristics, with a corresponding influence on the antimicrobial activity [Yang Wang, Jianbo Chen, Xin Zheng, Xiaoli Yang, Panpan M, Ying Cai, Bangzhi Zhang and Yuan Chena (2014). Design of novel analogues of short antimicrobial peptide anoplin with improved antimicrobial activity. J. Pept. Sci.; 20: 945-951. DOI 10.1002/psc.2705; Hyung-Sik Won, Min-Duk Seo, Seo-Jeong Jung, Sang-Jae Lee, Su-Jin Kang, Woo-Sung Son, Hyun-Jung Kim, Tae-Kyu Park, Sung-Jean Park, and Bong-Jin Lee, Structural Determinants for the Membrane Interaction of Novel Bioactive Undecapeptides Derived from Gaegurin 5. J. Med. Chem. 2006, 49, 4886-4895]. Hydrophobicity is known to affect both mammalian cell toxicity and antimicrobial activity. Hydrophobic residues facilitate interactions with fatty acyl chains. Relatively low hydrophobicity prevents binding to zwitterionic membranes found in mammalian cells, resulting in low toxicity. However, hydrophobicity is required for permeabilization of the bacterial membrane, but some studies have shown that beyond an optimal level of hydrophobicity, a further increase leads to a loss of antimicrobial activity and an increase in toxicity. Amphipathicity reflects the possibility of an amino acid sequence to form well-structured hydrophobic and hydrophilic domains on opposite faces. The Boman index was originally designated as the protein binding potential and was later renamed as the Boman index. This function calculates the sum of the solubility values for all residues in a sequence and, for normalization, is divided by the number of residues. The Boman index provides an overall estimate of the membrane binding potential of a peptide. The propensity of proteins or peptides for an (intrinsically) disordered conformation plays a key role in cellular regulation and signalling processes. Proteins and peptides under different biological conditions show marginal structural stability, and repeatedly unfold and fold *in vivo.* Indeed, numerous studies have shown that the effects of the denatured state, such as residual structure, excluded volume and intrinsic conformational propensities, play a key role in molecular recognition, allosteric signalling, folding, and stability [Peter Tompa, Eva Schad, Agnes Tantos and Lajos Kalmar (2015). Intrinsically disordered proteins: emerging interaction specialists. Current Opinion in Structural Biology, 35:49-59. doi.org/10.1016/j.sb1.2015.08.009].

Table 16

| | RiLK1 | HE10 |
|---|---|---|
| Half-life (sec) | 855.71 | 835.91 |
| Hydrophobicity | -0.56 | -0.36 |
| Hydropathicity | -1.12 | 0.23 |
| Amphipaticity | 1.35 | 0.98 |
| Hydrophilicity | 0.31 | 0.02 |
| Net charge | 5.0 | 4.0 |
| Boman index | 4.67 | 3.45 |
| Propensity for a disordered conformation | -0.61 | -0.09 |

Table 16 therefore clearly shows that the RiLK1 peptide of the invention and the HE10 peptide of the prior art exhibit considerable differences in terms of chemical-physical properties. It therefore appears that a 60% identity percentage (which should be considered low in any case) cannot in any way be considered predictive of a chemical-physical and therefore functional similarity between the two peptides.

This is further confirmed by the data in Table 17, which shows the same prediction, carried out with the same algorithms, on 4 other AMP peptides of the invention. These 4 peptides also exhibit values of hydrophobicity, hydropathicity, amphiphilicity, hydrophilicity, net charge, Boman index and propensity for a disordered conformation similar to each other and to RiLK1 but completely different compared to HE10.

Table 17

| | RiLK1 | RiLK2 | RiLK4 | RiLK7 | RiLK23 | HE10 |
|---|---|---|---|---|---|---|
| Half-life (sec) | 855.71 | 855.71 | 920.11 | 855.71 | 920.11 | 835.91 |
| Hydrophobicity | -0.56 | -0.56 | -0.49 | -0.56 | -0.49 | -0.36 |
| Hydropathicity | -1.12 | -1.12 | -1.06 | -1.12 | -1.06 | 0.23 |
| Amphipaticity | 1.35 | | | | | 0.98 |
| Hydrophilicity | 0.31 | 0.31 | 0.31 | 0.31 | 0.31 | 0.02 |
| Net charge | 5.0 | 5.0 | 5.0 | 5.0 | 5.0 | 4.0 |
| Boman index | 4.66 | 4.66 | 5.6 | 4.66 | 3.73 | 3.45 |
| Propensity for a disordered conformation | -0.61 | -0.61 | -0.62 | -0.61 | -0.59 | -0.09 |

The inventors also found that the presence of more than one tryptophan (W) residue - a feature common to all the AMP peptides of the invention - plays an important role in determining the antimicrobial properties, as it represents an advantageous and distinctive feature of the interface region between the lipid bilayers. It is also known that the side chains of tryptophan residues are involved in peptide folding in aqueous solution [David I. Chan, Elmar J. Prenner, Hans J. Vogel (2006). Tryptophan- and arginine-rich antimicrobial peptides: Structures and mechanisms of action. Biochimica et Biophysica Acta 1758; 1184-1202 doi:10.1016/j.bbamem.2006.04.006]. Considering not only the effects of the primary structure but also those of the secondary structure on the antimicrobial activity, the ability to assume an amphipathic structure is a functionally important feature for AMP incorporation into bacterial membranes [Marlon H. Cardoso, Karen G.N. Oshiro, Samilla B. Rezende, Elizabete S. Candido, Octávio L. Franco. The Structure/Function Relationship in Antimicrobial Peptides: What Can we Obtain From Structural Data? Advances in Protein Chemistry and Structural Biology · February 2018, DOI: 10.1016/bs.apcsb.2018.01.008]. All the AMP peptides of the invention have at least a second tryptophan residue inserted in the central part of the sequence, which has the purpose of enhancing the amphipathicity of the helix.

NMR analysis of the RiLK1 peptide of the invention in a micellar environment (SDS) has clearly demonstrated the existence of a well-defined structure in the helical arrangement, which places the positively charged side chains and the hydrophobic side chains on opposite sides. In this arrangement, all charged side chains are iso-oriented and prone to interact with the negatively charged surface of the SDS micelles.

Furthermore, the two side chains of the tryptophan residues, which are present in all the AMP peptides of the present invention in the W-X-X-X-W motif that characterizes them, are located at a suitable distance for mutual stacking, thus contributing to the stabilization of the helix.

The HE10 decapeptide described in WO2015038339 and all the 10 amino acid-long analogues thereof, on the other hand, do <u>not</u> contain the W-X-X-X-W motif which characterizes the AMP peptides of the invention and which, for the reasons illustrated above, represents a functionally important feature for the bactericidal activity.

Therefore, the AMP peptides of the invention are not similar to the HE10 peptide of the prior art neither in structural nor in functional terms.

## Claims

1. An antimicrobial peptide consisting of the amino acid sequence having the following general formula:

$$X_1LX_2WVX_3IWX_4X_5$$

   wherein $X_1$, $X_2$, $X_3$, $X_4$ and $X_5$ are independently selected from K and R and wherein each amino acid is independently in D or L configuration,
   or a salt or a solvate thereof.

2. The antimicrobial peptide according to claim 1, wherein at least one of $X_1$, $X_3$, $X_4$ and $X_5$ has the meaning of R; more preferably, $X_4$ and $X_5$ have the meaning of R; still more preferably, $X_3$, $X_4$ and $X_5$ have the meaning of R; even more preferably, $X_1$, $X_3$, $X_4$ and $X_5$ have the meaning of R.

3. The antimicrobial peptide according to claim 1 or 2, wherein $X_2$ has the meaning of K.

4. The antimicrobial peptide according to any one of claims 1 to 3, which is selected from the group consisting of $RLX_2WVRIWX_4X_5$, $RLX_2WVRIWRX_5$, $RLX_2WVRIWX_4K$, $RLKX_2WVRIWKK$, $X_1LKWVX_3IWRR$, $X_1LKWVRIWRR$, $RLKWVX_3IWRR$, $X_1LRWVX_3IWKK$, $X_1LRWVKIWKK$ and $KLRWVX_3IWKK$,
   wherein $X_1$, $X_2$, $X_3$, $X_4$ and $X_5$ are independently selected from K and R and wherein each amino acid is independently in D or L configuration.

5. The antimicrobial peptide according to claim 1, which is selected from the group consisting of RLKWVRIWRR (SEQ ID NO: 1), KLRWVRIWRR (SEQ ID NO: 2), RLRWVRIWRR (SEQ ID NO: 3), KLKWVRIWRR (SEQ ID NO: 4), RLKWVKIWRR (SEQ ID NO: 5), KLRWVKIWRR (SEQ ID NO: 6), RLRWVKIWRR (SEQ ID NO: 7), KLKWVKIWRR (SEQ ID NO: 8), RLKWVRIWKR (SEQ ID NO: 9), KLRWVRIWKR (SEQ ID NO: 10), RLRWVRIWKR (SEQ ID NO: 11), KLKWVRIWKR (SEQ ID NO: 12), RLKWVKIWKR (SEQ ID NO: 13), KLRWVKIWKR (SEQ ID NO: 14), RLRWVKIWKR (SEQ ID NO: 15), KLKWVKIWKR (SEQ ID NO: 16), RLKWVRIWRK (SEQ ID NO: 17), KLRWVRIWRK (SEQ ID NO: 18), RLRWVRIWRK (SEQ ID NO: 19), KLKWVRIWRK (SEQ ID NO: 20), RLKWVKIWRK (SEQ ID NO: 21), KLRWVKIWRK (SEQ ID NO: 22), RLRWVKIWRK (SEQ ID NO: 23), KLKWVKIWRK (SEQ ID NO: 24), RLKWVRIWKK (SEQ ID NO: 25), KLRWVRIWKK (SEQ ID NO: 26), RLRWVRIWKK (SEQ ID NO: 27), KLKWVRIWKK (SEQ ID NO: 28), RLKWVKIWKK (SEQ ID NO: 29), KLRWVKIWKK (SEQ ID NO: 30), RLRWVKIWKK (SEQ ID NO: 31) and KLKWVKIWKK (SEQ ID NO: 32),
   wherein each amino acid is independently in D or L configuration.

6. The antimicrobial peptide according to any one of claims 1 to 5, for use as a medicament, preferably in the therapeutic treatment of an infection caused by Gram negative bacteria, Gram positive bacteria, fungi, yeasts and/or viruses.

7. A pharmaceutical formulation comprising an antimicrobial peptide according to any one of claims 1 to 5 and at least one pharmaceutically acceptable excipient and/or carrier.

8. An antimicrobial composition comprising a carrier and at least one antimicrobial peptide according to any one of claims 1 to 5, optionally in combination with at least one further antimicrobial peptide according to any one of claims 1 to 5 and/or with at least one further antimicrobial agent.

9. The use of an antimicrobial peptide according to any one of claims 1 to 5 as an antimicrobial agent for the prevention of contamination of an article by and/or for the decontamination of an article from Gram negative bacteria, Gram positive bacteria, fungi, yeasts and/or viruses, wherein said article is preferably selected from a food, a material, a container or a tool.

10. An article bearing an antimicrobial peptide covalently bonded to a surface thereof, or bearing an antimicrobial peptide contained in a coating adhered to said surface, wherein said antimicrobial peptide is an antimicrobial peptide according to any one of claims 1 to 5 and wherein said article is preferably selected from a food, a material, a container and a tool.

**Patentansprüche**

1. Antimikrobielles Peptid, bestehend aus der Aminosäuresequenz mit der folgenden allgemeinen Formel:

$$X_1LX_2WVX_3IWX_4X_5$$

worin $X_1$, $X_2$, $X_3$, $X_4$ und $X_5$ unabhängig voneinander aus K und R ausgewählt sind und worin jede Aminosäure unabhängig voneinander in D- oder L-Konfiguration vorliegt, oder ein Salz oder ein Solvat davon.

2. Antimikrobielles Peptid nach Anspruch 1, worin mindestens einer von $X_1$, $X_3$, $X_4$ und $X_5$ die Bedeutung von R hat; stärker bevorzugt haben $X_4$ und $X_5$ die Bedeutung von R; noch stärker bevorzugt haben $X_3$, $X_4$ und $X_5$ die Bedeutung von R; am stärksten bevorzugt haben $X_1$, $X_3$, $X_4$ und $X_5$ die Bedeutung von R.

3. Antimikrobielles Peptid nach Anspruch 1 oder 2, worin $X_2$ die Bedeutung von K hat.

4. Antimikrobielles Peptid nach einem der Ansprüche 1 bis 3, ausgewählt aus der Gruppe bestehend aus $RLX_2WVRIWX_4X_5$, $RLX_2WVRIWRX_5$, $RLX_2WVRIWX_4K$, $RLKX_2WVRIWKK$, $X_1LKWVX_3IWRR$, $X_1LKWVRIWRR$, $RLKWVX_3IWRR$, $X_1LRWVX_3IWKK$, $X_1LRWVKIWKK$ und $KLRWVX_3IWKK$, worin $X_1$, $X_2$, $X_3$, $X_4$ und $X_5$ unabhängig voneinander aus K und R ausgewählt sind und worin jede Aminosäure unabhängig voneinander in D- oder L-Konfiguration vorliegt.

5. Antimikrobielles Peptid nach Anspruch 1, ausgewählt aus der Gruppe bestehend aus RLKWVRIWRR (SEQ ID NO: 1), KLRWVRIWRR (SEQ ID NO: 2), RLRWVRIWRR (SEQ ID NO: 3), KLKWVRIWRR (SEQ ID NO: 4), RLKWVKIWRR (SEQ ID NO: 5), KLRWVKIWRR (SEQ ID NO: 6), RLRWVKIWRR (SEQ ID NO: 7), KLKWVKIWRR (SEQ ID NO: 8), RLKWVRIWKR (SEQ ID NO: 9), KLRWVRIWKR (SEQ ID NO: 10), RLRWVRIWKR (SEQ ID NO: 11), KLKWVRIWKR (SEQ ID NO: 12), RLKWVKIWKR (SEQ ID NO: 13), KLRWVKIWKR (SEQ ID NO: 14), RLRWVKIWKR (SEQ ID NO: 15), KLKWVKIWKR (SEQ ID NO: 16), RLKWVRIWRK (SEQ ID NO: 17), KLRWVRIWRK (SEQ ID NO: 18), RLRWVRIWRK (SEQ ID NO: 19), KLKWVRIWRK (SEQ ID NO: 20), RLKWVKIWRK (SEQ ID NO: 21), KLRWVKIWRK (SEQ ID NO: 22), RLRWVKIWRK (SEQ ID NO: 23), KLKWVKIWRK (SEQ ID NO: 24), RLKWVRIWKK (SEQ ID NO: 25), KLRWVRIWKK (SEQ ID NO: 26), RLRWVRIWKK (SEQ ID NO: 27), KLKWVRIWKK (SEQ ID NO: 28), RLKWVKIWKK (SEQ ID NO: 29), KLRWVKIWKK (SEQ ID NO: 30), RLRWVKIWKK (SEQ ID NO: 31) und KLKWVKIWKK (SEQ ID NO: 32), worin jede Aminosäure unabhängig voneinander in D- oder L-Konfiguration vorliegt.

6. Antimikrobielles Peptid nach einem der Ansprüche 1 bis 5 zur Verwendung als Arzneimittel, bevorzugt bei der therapeutischen Behandlung einer durch Gram-negative Bakterien, Gram-positive Bakterien, Pilze, Hefen und/oder Viren verursachten Infektion.

7. Pharmazeutische Formulierung, umfassend ein antimikrobielles Peptid nach einem der Ansprüche 1 bis 5 und mindestens einen pharmazeutisch akzeptablen Hilfsstoff und/oder Träger.

8. Antimikrobielle Zusammensetzung, umfassend einen Träger und mindestens ein antimikrobielles Peptid nach einem der Ansprüche 1 bis 5, gegebenenfalls in Kombination mit mindestens einem weiteren antimikrobiellen Peptid nach einem der Ansprüche 1 bis 5 und/oder mit mindestens einem weiteren antimikrobiellen Wirkstoff.

9. Verwendung eines antimikrobiellen Peptids nach einem der Ansprüche 1 bis 5 als antimikrobieller Wirkstoff zur Verhinderung der Kontamination eines Artikels durch Gram-negative Bakterien, Gram-positive Bakterien, Pilze, Hefen und/oder Viren und/oder zur Dekontamination eines Artikels von Gram-negativen Bakterien, Gram-positiven Bakterien, Pilzen, Hefen und/oder Viren, worin der genannte Artikel bevorzugt aus einem Lebensmittel, einem Material, einem Behälter oder einem Werkzeug ausgewählt ist.

10. Artikel, der ein antimikrobielles Peptid trägt, das kovalent an eine Oberfläche davon gebunden ist, oder der ein antimikrobielles Peptid trägt, das in einer an der genannten Oberfläche haftenden Beschichtung enthalten ist, worin das genannte antimikrobielle Peptid ein antimikrobielles Peptid nach einem der Ansprüche 1 bis 5 ist und worin der genannte Artikel bevorzugt aus einem Lebensmittel, einem Material, einem Behälter und einem Werkzeug ausgewählt ist.

**Revendications**

1. Peptide antimicrobien consistant en la séquence d'acides aminés ayant la formule générale suivante :

$$X_1LX_2WVX_3IWX_4X_5$$

dans laquelle $X_1$, $X_2$, $X_3$, $X_4$ et $X_5$ sont indépendamment sélectionnés parmi K et R et dans laquelle chaque acide aminé est indépendamment en configuration D ou L, ou un sel ou un solvate de celui-ci.

2. Peptide antimicrobien selon la revendication 1, dans lequel au moins l'un parmi $X_1$, $X_2$, $X_3$, $X_4$ et $X_5$ a la signification de R ; de préférence, $X_4$ et $X_5$ ont la signification de R ; plus préférablement encore, $X_3$, $X_4$ et $X_5$ ont la signification de R ; encore plus préférablement, $X_1$, $X_3$, $X_4$ et $X_5$ ont la signification de R.

3. Peptide antimicrobien selon la revendication 1 ou 2, dans lequel $X_2$ a la signification de K.

4. Peptide antimicrobien selon l'une quelconque des revendications 1 à 3, sélectionné dans le groupe consistant en $RLX_2WVRIWX_4X_5$, $RLX_2WVRIWRX_5$, $RLX_2WVRIWX_4K$, $RLKX_2WVRIWKK$, $X_1LKWVX_3IWRR$, $X_1LKWVRIWRR$, $RLKWVX_3IWRR$, $X_1LRWVX_3IWKK$, $X_1LRWVKIWKK$ et $KLRWVX_3IWKK$, dans lequel $X_1$, $X_2$, $X_3$, $X_4$ et $X_5$ sont indépendamment sélectionnés parmi K et R et dans lequel chaque acide aminé est indépendamment en configuration D ou L.

5. Peptide antimicrobien selon la revendication 1, sélectionné dans le groupe consistant en RLKWVRIWRR (SEQ ID NO : 1), KLRWVRIWRR (SEQ ID NO : 2), RLRWVRIWRR (SEQ ID NO : 3), KLKWVRIWRR (SEQ ID NO : 4), RLKWVKIWRR (SEQ ID NO : 5), KLRWVKIWRR (SEQ ID NO : 6), RLRWVKIWRR (SEQ ID NO : 7), KLKWVKIWRR (SEQ ID NO : 8), RLKWVRIWKR (SEQ ID NO : 9), KLRWVRIWKR (SEQ ID NO : 10), RLRWVRIWKR (SEQ ID NO : 11), KLKWVRIWKR (SEQ ID NO : 12), RLKWVKIWKR (SEQ ID NO : 13), KLRWVKIWKR (SEQ ID NO : 14), RLRWVKIWKR (SEQ ID NO : 15), KLKWVKIWKR (SEQ ID NO : 16), RLKWVRIWRK (SEQ ID NO : 17), KLRWVRIWRK (SEQ ID NO : 18), RLRWVRIWRK (SEQ ID NO : 19), KLKWVRIWRK (SEQ ID NO : 20), RLKWVKIWRK (SEQ ID NO : 21), KLRWVKIWRK (SEQ ID NO : 22), RLRWVKIWRK (SEQ ID NO : 23), KLKWVKIWRK (SEQ ID NO : 24), RLKWVRIWKK (SEQ ID NO : 25), KLRWVRIWKK (SEQ ID NO : 26), RLRWVRIWKK (SEQ ID NO : 27), KLKWVRIWKK (SEQ ID NO : 28), RLKWVKIWKK (SEQ ID NO : 29), KLRWVKIWKK (SEQ ID NO : 30), RLRWVKIWKK (SEQ ID NO : 31) et KLKWVKIWKK (SEQ ID NO : 32), dans lequel chaque acide aminé est indépendamment en configuration D ou L.

6. Peptide antimicrobien selon l'une quelconque des revendications 1 à 5, destiné à être utilisé comme médicament, de préférence dans le traitement thérapeutique d'une infection causée par des bactéries Gram négatives, des bactéries Gram positives, des champignons, des levures et/ou des virus.

7. Formulation pharmaceutique comprenant un peptide antimicrobien selon l'une quelconque des revendications 1 à 5 et au moins un excipient et/ou véhicule pharmaceutiquement acceptable.

8. Composition antimicrobienne comprenant un véhicule et au moins un peptide antimicrobien selon l'une quelconque des revendications 1 à 5, éventuellement en combinaison avec au moins un autre peptide antimicrobien selon l'une quelconque des revendications 1 à 5 et/ou avec au moins un autre agent antimicrobien.

9. Utilisation d'un peptide antimicrobien selon l'une quelconque des revendications 1 à 5 en tant qu'agent antimicrobien pour la prévention de la contamination d'un article par des bactéries Gram négatives, des bactéries Gram positives, des champignons, des levures et/ou des virus et/ou pour la décontamination d'un article de bactéries Gram négatives, de bactéries Gram positives, de champignons, de levures et/ou de virus, dans laquelle ledit article est de préférence sélectionné parmi un aliment, un matériau, un récipient ou un outil.

10. Article portant un peptide antimicrobien lié de manière covalente à une surface de celui-ci, ou portant un peptide antimicrobien contenu dans un revêtement adhérant à ladite surface, dans lequel ledit peptide antimicrobien est un peptide antimicrobien selon l'une quelconque des revendications 1 à 5 et dans lequel ledit article est de préférence sélectionné parmi un aliment, un matériau, un récipient et un outil.

FIG. 1

FIG.2

FIG. 3a

FIG. 3b

FIG.4

FIG. 5

FIG. 6

A

B

C

D

FIG. 7

FIG. 8

FIG. 9

A

B

C

D

FIG. 10

(1)  (2)  (3)

FIG. 11a

(1)  (2)  (3)

FIG. 11b

FIG. 11c

FIG. 11d

FIG. 12

1                          2

*Aspergillus brasiliensis*

*Candida albicans*

FIG. 13

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015038339 A

- WO 2019012158 A


**Non-patent literature cited in the description**

- Innate immunity. **ALBERTS B** ; **JOHNSON A** ; **LEWIS J et al.** Molecular Biology of the Cell. Garland Science, 2002
- **WANG, G** ; **LI, X** ; **WANG, Z.** APD3: the antimicrobial peptide database as a tool for research and education. *Nucleic Acids Research*, 2016, vol. 44, D1087-D1093
- **JOHANNES KOEHBACH** ; **DAVID J. CRAIK**. The Vast Structural Diversity of Antimicrobial Peptides. *Trends in Pharmacological Sciences*, July 2019, vol. 40 (7)
- **RALF MIKUT** ; **SERGE RUDEN** ; **MARKUS REISCHL** ; **FRANK BREITLING** ; **RUDOLF VOLKMER** ; **KAI HILPERT**. Improving short antimicrobial peptides despite elusive rules for activity. *Biochimica et Biophysica Acta*, 2016, vol. 1858, 1024-1033
- **SUKRITA PUNYAUPPA-PATH** ; **PARICHAT PHUMKHACHORN** ; **PONGSAK RATTANACHAIKUNSOPON**. NISIN: PRODUCTION AND MECHANISM OF ANTIMICROBIAL ACTION. *Int J Cur Res Rev*, January 2015, vol. 7 (2)
- **H BROTZ** ; **HG SAHL**. New insights into the mechanism of action of lantibiotics - diverse biological effects by binding to the same molecular target. *Journal of Antimicrobial Chemotherapy*, 2000, vol. 46, 1-6
- **MAHDI SHAHMIRI** ; **MARTA ENCISO** ; **CHRISTOPHER G. ADDA** ; **BRIAN J. SMITH** ; **MATTHEW A. PERUGINI** ; **ADAM MECHLER**. Membrane Core-Specific Antimicrobial Action of Cathelicidin LL-37 Peptide Switches Between Pore and Nanofibre Formation. *Scientific Reports*, 2016, vol. 6
- Human Antimicrobial Peptides as Therapeutics for Viral Infections. *Viruses*, 2019, vol. 11, 704
- **E. W. MARTIN**. Remington's Pharmaceutical Sciences
- **WANG, Y.J.** ; **HANSON, M. A.** Journal of Parenteral Science and Technology. *Technical Report No. 10*

- **HEGEMANN, DIRK** ; **HERWIG BRUNNER** ; **CHRISTIAN OEHR**. Plasma treatment of polymers for surface and adhesion improvement. *Nuclear instruments and methods in physics research section B: Beam interactions with materials and atoms*, 2003, vol. 208, 281-286
- **WHITMORE L** ; **WALLACE BA**. *Nucleic Acids Research*, 2004, vol. 32, 668-673
- **WHITMORE L** ; **WALLACE BA**. *Biopolymers*, 2008, vol. 89, 392-400
- **LOBLEY et al.** *Bioinformatics*, 2002, vol. 18, 211-212
- **SREEREMA N** ; **WOODY RW**. *Analytical Biochemistry*, 1993, vol. 287, 252-260
- **ABDUL-GADER A et al.** *Bioinformatics*, 2011, vol. 27, 1630-1636
- **MICSONAI A. et al.** *Nucleic Acids Res.*, 2018, vol. 46, W315-W322
- **MICSONAI A. et al.** *Proc Natl Acad Sci U S A*, 2015, vol. 112, E3095-E3103
- **WANG HX** ; **NG TB**. *Peptides*, 2003, vol. 24, 969-972
- **BILIKOVA et al.** *Peptides*, 2015, vol. 68, 190-196
- **BÍLIKOVA et al.** *Peptides*, 2015, vol. 68, 190-196
- **MCWILLIAM H** ; **LI W** ; **ULUDAG M** ; **SQUIZZATO S** ; **PARK YM** ; **BUSO N** ; **COWLEY AP** ; **LOPEZ R**. Analysis Tool Web Services from the EMBL-EBI. *Nucleic acids research*, July 2013, vol. 41, W597-600
- **KOONIN EV** ; **GALPERIN MY**. Principles and Methods of Sequence Analysis - Sequence - Evolution - Function - NCBI Bookshelf
- Sequence - Evolution - Function: Computational Approaches in Comparative Genomics. Kluwer Academic, 2003
- **HANS G. BOMAN**. Peptide antibiotics and their role in innate immunity. *Annu. Rev. Immunol*, 1995, vol. 13, 61-92

- **MARKÉTA PAZDERKOVÁ** ; **PETR MALO** ; **VLASTIMIL ZÍMA** ; **KATERINA HOFBAUEROVÁ** ; **VLADIMÍR KOPECKÝ JR.** ; **EVA KOCIŠOVÁ** ; **TOMÁŠ PAZDERKA** ; **VÁCLAV CEROVSKÝ** ; **LUCIE BEDNÁROVÁ**. Interaction of Halictine-Related Antimicrobial Peptides with Membrane Models. *Int. J. Mol. Sci*, 2019, vol. 20, 631
- **IGOR ZELEZETSKY** ; **ALESSANDRO TOSSI**. Alpha-helical antimicrobial peptides-Using a sequence template to guide structure-activity relationship studies. *Biochimica et Biophysica Acta*, 2006, vol. 1758, 1436-1449
- **YANG WANG** ; **JIANBO CHEN** ; **XIN ZHENG** ; **XIAOLI YANG** ; **PANPAN M** ; **YING CAI** ; **BANGZHI ZHANG** ; **YUAN CHENA**. Design of novel analogues of short antimicrobial peptide anoplin with improved antimicrobial activity. *J. Pept. Sci*, 2014, vol. 20, 945-951
- **HYUNG-SIK WON** ; **MIN-DUK SEO** ; **SEO-JEONG JUNG** ; **SANG-JAE LEE** ; **SU-JIN KANG** ; **WOO-SUNG SON** ; **HYUN-JUNG KIM** ; **TAE-KYU PARK** ; **SUNG-JEAN PARK** ; **BONG-JIN LEE**. Structural Determinants for the Membrane Interaction of Novel Bioactive Undecapeptides Derived from Gaegurin 5. *J. Med. Chem*, 2006, vol. 49, 4886-4895
- **PETER TOMPA** ; **EVA SCHAD** ; **AGNES TANTOS** ; **LAJOS KALMAR**. Intrinsically disordered proteins: emerging interaction specialists. *Current Opinion in Structural Biology*, 2015, vol. 35, 49-59
- **DAVID I. CHAN** ; **ELMAR J. PRENNER** ; **HANS J. VOGEL**. Tryptophan- and arginine-rich antimicrobial peptides: Structures and mechanisms of action. *Biochimica et Biophysica Acta*, 2006, vol. 1758, 1184-1202
- **MARLON H. CARDOSO** ; **KAREN G.N. OSHIRO** ; **SAMILLA B. REZENDE** ; **ELIZABETE S. CANDIDO** ; **OCTÁVIO L. FRANCO**. The Structure/Function Relationship in Antimicrobial Peptides: What Can we Obtain From Structural Data?. *Advances in Protein Chemistry and Structural Biolo*, February 2018